(19) Europäisches Patentamt
European Patent Office
Office européen des brevets

(11) EP 1 477 513 B1

(12) # EUROPÄISCHE PATENTSCHRIFT

(45) Veröffentlichungstag und Bekanntmachung des Hinweises auf die Patenterteilung:
**15.06.2005 Patentblatt 2005/24**

(51) Int Cl.7: **C08G 77/46**, C08G 77/388, D06M 15/647

(21) Anmeldenummer: **04009000.3**

(22) Anmeldetag: **15.04.2004**

(54) **Ammoniumfunktionelle Polysiloxancopolymere**

Ammonium-group containing polysiloxane copolymers

Copolymères organosiliciques contenant des groupes ammonium.

(84) Benannte Vertragsstaaten:
**BE DE FR GB NL PL**

(30) Priorität: **08.05.2003 DE 10320631**

(43) Veröffentlichungstag der Anmeldung:
**17.11.2004 Patentblatt 2004/47**

(73) Patentinhaber: **Wacker-Chemie GmbH**
**81737 München (DE)**

(72) Erfinder:
- **Hohberg, Thomas, Dr.**
  **84489 Burghausen (DE)**
- **Ochs, Christian, Dr.**
  **84489 Burghausen (DE)**

(74) Vertreter: **Fritz, Helmut, Dr. et al**
**Wacker-Chemie GmbH,**
**Zentralabteilung Patente,**
**Marken und Lizenzen,**
**Hanns-Seidel-Platz 4**
**81737 München (DE)**

(56) Entgegenhaltungen:
**WO-A-02/10257** **WO-A-03/035721**

- **PATENT ABSTRACTS OF JAPAN Bd. 2003, Nr. 02, 5. Februar 2003 (2003-02-05) & JP 2002 308991 A (SHIN ETSU CHEM CO LTD), 23. Oktober 2002 (2002-10-23)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 477 513 B1

## Beschreibung

Die Erfindung betrifft Organosiliciumverbindungen, bei denen kationische Quatgruppen und Polyethergruppen über eine Aminooder Ammoniumgruppe an den Siloxankörper gebunden sind, und ein Verfahren zu deren Herstellung.

Ternäre Polysiloxansysteme, bestehend aus Polysiloxaneinheiten, Polyethergruppen und kationischen Quatgruppen, sind von besonderem Interesse für die Anwendung als Textilweichmacher. Die beschriebenen Synthesen sind jedoch ausschließlich mehrstufige, sehr zeitintensive Syntheseverfahren, da die verwendeten Edukte oftmals technisch nicht verfügbar sind und explizit hergestellt werden müssen. Die damit verknüpften, schlechten Raum-Zeit-Ausbeuten machen die bestehenden Syntheseverfahren kostenungünstig und unpraktikabel.
Die bekannten, oben genannten ternären Polysiloxansysteme lassen sich in 4 verschiedene Produktklassen einteilen: A) streng kammförmig modifizierte Siloxanpolymere, in denen eine Polyethergruppe als Bindeglied zwischen Siloxangerüst und kationischer Quatgruppe, bzw. umgekehrt, d.h. in denen die kationische Quatgruppe als Bindeglied zwischen Siloxangerüst und Polyethergruppe fungiert, sind z.B. beschrieben in US 6,030,675; B) lineare Siloxancopolymere mit terminalen kationischen Quatgruppen und Polyethergruppen, wobei die Polyethergruppe auch Bindeglied zu einem weiteren Siloxanblock mit terminaler Quatgruppe sein kann, sind z.B. beschrieben in EP 1000959 A; C) Polymere, in denen die kationischen Quatgruppen einen verbrückenden Teil zwischen 2 Siloxanblöcken des Polymers darstellen, wobei zusätzliche Polyethergruppen als 2-wertige Bindeglieder in der Polymerhauptkette liegen können, oder das Siloxangerüst bzw. die kationische Quatgruppe kammförmig Polyethermodifiziert ist sind z.B. beschrieben in WO 02/10257 A1; D) Polysiloxane, welche end- und/oder seitenständig mit voneinander unabhängigen Polyethergruppen und kationischen Quatgruppen modifiziert sind, sind z.B. beschrieben in US 6,313,256 A.

Gegenstand der Erfindung sind Copolymere (Q) der allgemeinen Formel (I)

$$A\text{-}B\text{-}(D\text{-}B)_o\text{-}A \qquad (I),$$

worin,

**A** einen Rest $-R^1$ oder $-OR^1$ oder einen einwertigen Rest der allgemeinen Formel (II),

$$-R-[(NH_aE_bF_c)^{(a+2b+c-1)+}-R]_g-(NH_dE_eF_f)^{(d+2e+f-2)+} \quad [g(a+2b+c-1)+d+2e+f-2]\ X^- \qquad (II),$$

**$R^1$** ein Wasserstoffatom oder einen einwertigen Kohlenwasserstoffrest mit 1 bis 100 Kohlenstoffatomen, der gegebenenfalls durch die Atome N, O, P, B, Si, S unterbrochen oder substituiert sein kann, oder Einheiten -C(O)-, -C(O)O-, -C(O)$NR^9$-, -$NR^9$-, -O-, -S-, =N-, ≡N enthalten kann, oder durch Gruppen -$NR^9$-, -OH, -SH substituiert sein kann,

**R** einen zweiwertigen Kohlenwasserstoffrest mit 1 bis 50 Kohlenstoffatomen, der gegebenenfalls durch die Atome N, O, P, B, Si, S unterbrochen oder substituiert sein kann, oder Einheiten -C(O)-, -C(O)O-, -C(O)$NR^9$-, -$NR^9$-, -O-, -S-, =N- enthalten kann,

**$R^9$** ein Wasserstoffatom oder einen einwertigen Kohlenwasserstoffrest mit 1 bis 18 Kohlenstoffatomen, der gegebenenfalls durch die Atome N, O, P, B, Si, S unterbrochen oder oder durch Gruppen -OH, -SH substituiert sein kann,

**a, b** und **c** ganze Zahlen von 0 bis 2, wobei die Summe aus **a+b+c** gleich 1 oder 2 ist,

**d, e** und **f** ganze Zahlen von 0 bis 3, wobei die Summe aus **d+e+f** gleich 2 oder 3 ist,

**g** eine ganze Zahl von 0 bis 10,

**E** einen Rest der allgemeinen Formel (III)

$$-R^2\text{-}NR^3{}_3{}^+ \qquad (III),$$

**$R^2$** einen zweiwertigen Kohlenwasserstoffrest mit 1 bis 10 Kohlenstoffatomen, der gegebenenfalls durch die Atome N, O, P, B, Si, S unterbrochen oder substituiert sein kann, oder Einheiten -C(O)-, -C(O)O-, -C(O)$NR^9$-, -$NR^9$-, -O-, -S-, =N-, =N enthalten kann, oder durch Gruppen -$NR^9$-, -OH, -SH substituiert sein kann,

**$R^3$** einen einwertigen Kohlenwasserstoffrest mit 1 bis 20 Kohlenstoffatomen, der gegebenenfalls durch die

Atome N, O, P, B, Si, S unterbrochen oder substituiert sein kann, oder Einheiten -C(O)-, -C(O)O-, --C(O)$NR^9$-, -$NR^9$-, -O-, -S-, =N-, ≡N enthalten kann, oder durch Gruppen -$NR^9$-, -OH, -SH substituiert sein kann,

**$X^-$** ein anorganisches oder organisches Anion,

**F** einen Rest der allgemeinen Formeln (IV) oder (V)

$$-CH_2-CHR^4-C(O)-G-(R^2-G)_h-[CH_2CH_2O]_i-[C_3H_6O]_j-[(CH_2)_4O]_k-(R^2-G)_h-R^5 \quad (IV),$$

$$-CH_2-CH(OH)-CH_2-G-(R^2-G)_h-[CH_2CH_2O]_i-[C_3H_6O]_j-[(CH_2)_4O]_k-(R^2-G)_h-R^5 \quad (V),$$

**$R^4$** ein Wasserstoffatom oder Methylrest,

**$R^5$** ein Wasserstoffatom, ein einwertiger, verzweigter oder unverzweigter Kohlenwasserstoffrest mit 1 bis 10 Kohlenstoffatomen oder eine Einheit ausgewählt aus der Gruppe $CH_2=CR^4-C(O)-$, $(R^1)_2N-CH_2-CHR^4-C(O)-$, $R^4-CH_2-C(O)-$, $HO-CH_2-CH(OH)-CH_2-$ oder

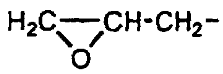

,

**G** eine zweiwertige Gruppe -O- oder -$NR^9$-,

**h** den Wert 0 oder 1,

**i, j** und **k** jeweils eine ganze Zahl von 0 bis 200,

**B** einen zweiwertigen Rest, aufgebaut aus Einheiten, die ausgewählt werden aus den allgemeinen Formeln (VIa), (VIb), (VIc), (VId) und **A**

$$(SiR^6{}_2O_{2/2})_1 \quad (VIa),$$

$$(SiR^6R^7O_{2/2})_m \quad (VIb),$$

$$(-SiR^6{}_2-)_{p+1} \quad (VIc),$$

$$(SiO_{3/2})_p \quad (VId),$$

**$R^6$** ein Wasserstoffatom, -$OR^1$, oder einen einwertigen Alkylrest mit 1 bis 200 Kohlenstoffatomen, der substituiert sein kann durch Halogenatome, Carboxy-, Epoxy-, Hydroxy- oder Polyethergruppen und gegebenenfalls durch die Einheiten -C(O)-, -C(O)O-, -C(O)$NR^9$-, -$NR^9$-, -O-, -S-, unterbrochen sein kann, bedeutet,

**$R^7$** ein einwertiger Rest der allgemeinen Formel (II),

**l** und m eine ganze Zahl von 0 bis 5000,

**p** eine ganze Zahl von 0 bis 500,

**D** einen zweiwertigen Rest der allgemeinen Formel (VIII),

$$-\{[R-(NH_aE_bF_c)]_n-J-[(NH_aE_bF_c)-R]_n\}-^{[2n(a+2b+c)-4]+} \quad [2n(a+2b+c)-4]\ X^- \quad (VIII),$$

**n** eine ganze Zahl von 1 bis 10,

**J** einen zweiwertigen Rest der allgemeinen Formeln (IX) oder (X),

$$-CH_2-CHR^4-C(O)-G-(R^2-G)_h-[CH_2CH_2O]_i-[C_3H_6O]_j-[(CH_2)_4O]_k-(R^2-G)_h-$$

$$C(O)\text{-}CHR^4\text{-}CH_2\text{-} \qquad (IX),$$

$$\text{-}CH_2\text{-}CH(OH)\text{-}CH_2\text{-}G\text{-}(R^2\text{-}G)_h\text{-}[CH_2CH_2O]_i\text{-}[C_3H_6O]_j\text{-}[(CH_2)_4O]_k\text{-}(R^2\text{-}G)_h\text{-}CH_2\text{-}CH(OH)\text{-}CH_2\text{-} \qquad (X),$$

**o** eine ganze Zahl ≥ 0 bedeuten,

mit der Maßgabe, daß die Copolymere der allgemeinen Formel (I) mindestens je einen Rest **E** und **F** enthalten.

Im Unterschied zu den bekannten Strukturen sind in den Copolymeren (Q) die kationische Quatgruppe und die Polyethergruppe über eine Amino- oder Ammoniumgruppe an den Siloxankörper gebunden. Die Quatgruppe wirkt dabei nicht verbrückend, während der Polyetherrest als Bindeglied zwischen 2 Aminosiloxankörpern fungieren kann.

Der Aufbau der Copolymere (Q) kann auf einfache Weise in einem Eintopfverfahren erfolgen. Die Synthesen verlaufen nahezu quantitativ und sind wegen guter Raum-Zeit-Ausbeuten kostengünstiger als alle Verfahren, die zu den bekannten Strukturen führen. Darüber hinaus sind alle Rohstoffe kommerziell in großen Mengen verfügbar und erfordern keinen zusätzlichen Syntheseaufwand. Die erhaltenen Copolymere (Q) können, in Abhängigkeit von der gewählten Stöchiometrie wasserlöslich oder selbstemulgierend (sog. "Selbstemulgierende Systeme") eingestellt werden, d.h. sie erfordern für die Emulgierung keine weiteren Hilfsmittel. Die Copolymere (Q) können zur Behandlung von textilen Flächengebilden, Textilfasern und Leder, als Additive in Beschichtungen und Lacken, als Zusätze in kosmetischen Formulierungen und als oberflächenaktive Mittel verwendet werden. Sie weisen insbesondere hervorragende Eigenschaften als Textilweichmacher auf, die denen gewöhnlicher Amino-Glykolöle überlegen sind.

Die Copolymere (Q) sind lineare oder verzweigte Aminopolysiloxane, deren α, ω- und/oder seitenständige Aminogruppen sowohl mit Polyethergruppen als auch mit kationischen Quatgruppen modifiziert sind, wobei die Aminogruppen der Aminopolysiloxane als quartäre Ammoniumgruppen vorliegen können.

Die Copolymere (Q) besitzen vorzugsweise ein durchschnittliches Molekulargewicht Mn von mindestens 500 g/mol, besonders bevorzugt mindestens 1000 g/mol und vorzugsweise höchstens 1 000 000 g/mol, besonders bevorzugt höchstens 500 000 g/mol.

Die Copolymere (Q) besitzen vorzugsweise eine Viskosität von mindestens 10 $mm^2/s$, insbesondere mindestens 15 $mm^2/s$, bis vorzugsweise fest bei 25°C.

Die Copolymere (Q) weisen vorzugsweise eine Aminzahl von mindestens 0,001, insbesondere mindestens 0,01, und vorzugsweise höchstens 9, besonders bevorzugt höchstens 6 auf. Die Aminzahl bezeichnet die Anzahl der mL 1-n-HCl, die zum Neutralisieren von 1 g Copolymer erforderlich sind.

Vorzugsweise besitzen a, b und c den Wert 0 oder 1.

Vorzugsweise beträgt die Summe aus a+b+c den Wert 1.

Vorzugsweise besitzen d, e und f den Wert 0, 1 oder 2.

Vorzugsweise beträgt die Summe aus d+e+f 2, und die Summe aus a+b+c+d+e+f ≤ 5, insbesondere 3 oder 4.

Vorzugsweise besitzt g einen Wert von höchstens 5. Besonders bevorzugt besitzt g den Wert 0 oder 1.

Vorzugsweise besitzt h den Wert 0.

Vorzugsweise besitzen i, j und k jeweils den Wert von höchstens 100, besonders bevorzugt von höchstens 50.

Vorzugsweise betragen 1 bzw. m höchstens 1000, besonders bevorzugt höchstens 500.

Vorzugsweise besitzt p einen Wert von höchstens 100, besonders bevorzugt höchstens 20.

Vorzugsweise besitzt n einen Wert von höchstens 5. Besonders bevorzugt besitzt n den Wert 1 oder 2, insbesondere den Wert 1. Vorzugsweise beträgt o höchstens 1000, insbesondere höchstens 100.

Vorzugsweise ist **R** ein zweiwertiger Kohlenwasserstoffrest mit 1 bis 10 Kohlenstoffatomen, der gegebenenfalls durch Atome N und O unterbrochen sein kann. Besonders bevorzugt als **R** sind Reste der Formeln, $-CH_2-$, $-(CH_2)_2-$, $-(CH_2)_3-$, $-(CH_2)_2-NH-(CH_2)_3-$, wobei - $(CH_2)-$, $-(CH_2)_2-$ und $-(CH_2)_3-$ insbesondere bevorzugt sind.

Vorzugsweise ist $R^1$ ein Wasserstoffatom, ein gesättigter oder ungesättigter Alkylrest mit 1 bis 10 Kohlenstoffatomen und die Reste -C(O)H, $-C(O)CH_3$, $-C(O)CH_2-CH_3$ und $-CH_2-CH_2-C(O)-O-CH_2-CH_3$. Besonders bevorzugt sind Wasserstoffatom, Methyl-, Ethyl-, Propyl-, Cyclohexyl- und der Acetylrest.

Vorzugsweise ist $\mathbf{R^2}$ ein zweiwertiger, nicht unterbrochener Kohlenwasserstoffrest mit 1 bis 8 Kohlenstoffatomen und der Rest - $CH_2-CH(OH)-CH_2-$. Bevorzugte Beispiele für $\mathbf{R^2}$ sind lineare oder verzweigte Alkylenreste wie der Ethylen-, Propylen-, Butylenrest, Arylenreste wie der Phenylenrest, und der Rest $-CH_2-CH(OH)-CH_2-$.

Vorzugsweise ist $\mathbf{R^3}$ ein einwertiger, nicht unterbrochener Alkylrest. Besonders bevorzugt sind Methyl-, Ethyl-, Propyl-, Butyl-, Cyclohexyl-, Dodecyl- und der Octadecylrest. Vorzugsweise ist $\mathbf{R^4}$ ein Wasserstoffatom.

Vorzugsweise ist $\mathbf{R^5}$ ein Wasserstoffatom, ein gesättigter oder ungesättigter Alkylrest mit 1 bis 5 Kohlenstoffatomen

oder einer der Reste $-C(O)CH_3$, $CH_2=CH-C(O)-$, $(R^1)_2N-CH_2-CH_2-C(O)-$ und

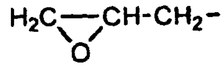

.

Besonders bevorzugt sind Wasserstoffatom, der Methyl-, Ethyl-, Butyl-, Acetyl- und Acryloylrest, sowie

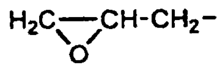

.

Beispiele für Rest **R⁶** sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neo-Pentyl-, tert.-Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest; Dodecylreste, wie der n-Dodecylrest; Octadecylreste, wie der n-Octadecyl-rest; Cycloalkylreste, wie der Cyclopentyl-, Cyclohexyl-, Cycloheptylrest und Methylcyclohexylreste; Alkenylreste, wie der Vinyl-, Allyl-, 3-Butenyl-, 5-Hexenyl-, 1-Propenyl- und 1-Pentenylrest; Alkinylreste, wie der Ethinyl-, Propargyl- und 1-Propinylrest; Arylreste, wie der Phenyl-, Naphthyl-, Anthrylund Phenanthrylrest; Alkarylreste, wie o-, m-, p-Tolylreste; Xylylreste und Ethylphenylreste; und Aralkylreste, wie der Benzylrest, der Phenylethylrest und der Phenylnonylrest, oder der Methoxy- oder Ethoxyrest.

Beispiele für substituierte Reste **$R^6$** sind Halogenalkylreste, wie der 3,3,3-Trifluor-n-propylrest, der 2,2,2,2',2',2'-Hexafluorisopropylrest, der Heptafluorisopropylrest und Halogenarylreste, wie der o-, m- und p-Chlorphenylrest, oder Alkylreste, substituiert mit Carboxy-, Epoxy-, Hydroxy- oder Polyethergruppen.

Vorzugsweise ist **$R^6$** ein Wasserstoffatom oder ein einwertiger Alkylrest mit 1 bis 10 Kohlenstoffatomen. Besonders bevorzugt ist der Methylrest.

Beispiele für den Rest $R^7$ sind

$-CH_2-\{N[-CH_2-CH(OH)-CH_2-NMe_3]F\}^+\ Cl^-$,
$-CH_2-\{N[-CH_2-CH(OH)-CH_2-NMe_3]_2F\}^{3+}\ 3Cl^-$,
$-(CH_2)_3-\{N[-CH_2-CH(OH)-CH_2-NMe_3]F\}^+\ Cl^-$,
$-(CH_2)_3-\{N[-CH_2-CH(OH)-CH_2-NMe_3]_2F\}^{3+}\ 3Cl^-$,
$-(CH_2)_3-NH-(CH_2)_2-\{N[-CH_2-CH(OH)-CH_2-NMe_3]F\}^+\ Cl^-$,
$-(CH_2)_3-\{N[-CH_2-CH(OH)-CH_2-NMe_3]-(CH_2)_2-NHF\}^+\ Cl^-$,
$-(CH_2)_3-NF-(CH_2)_2-\{NH[-CH_2-CH(OH)-CH_2-NMe_3]\}^+\ Cl^-$,
$-(CH_2)_3-\{N[-CH_2-CH(OH)-CH_2-NMe_3]-(CH_2)_2-NF_2\}^+\ Cl^-$,
$-(CH_2)_3-NF-(CH_2)_2-\{N[-CH_2-CH(OH)-CH_2-NMe_3]F\}^+\ Cl^-$,
$-(CH_2)_3-\{N[-CH_2-CH(OH)-CH_2-NMe_3]-(CH_2)_2-N[-CH_2-CH(OH)-CH_2-NMe_3]F\}^{2+}\ 2Cl^-$,
$-(CH_2)_3-NF-(CH_2)_2-\{N[-CH_2-CH(OH)-CH_2-NMe_3]_2\}^{2+}\ 2Cl^-$,
$-(CH_2)_3-\{N[-CH_2-CH(OH)-CH_2-NMe_3]_2-(CH_2)_2-NF_2\}^{3+}\ 3Cl^-$,
$-(CH_2)_3-\{N[-CH_2-CH(OH)-CH_2-NMe_3]F-(CH_2)_2-N[-CH_2-CH(OH)-CH_2-NMe_3]F\}^{3+}\ 3Cl^-$,
$-(CH_2)_3-\{N[-CH_2-CH(OH)-CH_2-NMe_3]-(CH_2)_2-N[-CH_2-CH(OH)-CH_2-NMe_3]F_2\}^{3+}\ 3Cl^-$,
$-(CH_2)_3-NF-(CH_2)_2-\{N[-CH_2-CH(OH)-CH_2-NMe_3]_2F\}^{3+}\ 3Cl^-$,
$-(CH_2)_3-NF-(CH_2)_2-N[-CH_2-CH(OH)-CH_2-NMe_3]_3\}^{4+}\ 4Cl^-$,
$-(CH_2)_3-\{N[-CH_2-CH(OH)-CH_2-NMe_3]-(CH_2)_2-N[-CH_2-CH(OH)-CH_2-NMe_3]_2F\}^{4+}\ 4Cl^-$,
$-(CH_2)_3-\{N[-CH_2-CH(OH)-CH_2-NMe_3]_2-(CH_2)_2-N[-CH_2-CH(OH)-CH_2-NMe_3]F\}^{4+}\ 4Cl^-$,
$-(CH_2)_3-\{N[-CH_2-CH(OH)-CH_2-NMe_3]F-(CH_2)_2-N[-CH_2-CH(OH)-CH_2-NMe_3]_2\}^{4+}\ 4Cl^-$,

wobei Me für Methylrest steht und sich durch Permutation mit den für Rest **F** genannten Beispielen eine Vielzahl weiterer, analoger Beispiele ergeben. Im folgenden sind exemplarisch einige repräsentative Beispiele für Rest **$R^7$** aufgeführt, um die Bandbreite der Copolymere (Q) zu demonstrieren.(Me = Methylrest):

$-CH_2-\{N[-CH_2-CH(OH)-CH_2-NMe_3][-CH_2-CH(OH)-CH_2-O-(C_2H_4O)_5-C_4H_9]\}^+\ Cl^-$,

$-CH_2-\{N[-CH_2-CH(OH)-CH_2-NMe_3][-CH_2-CH(OH)-CH_2-O-(C_3H_6O)_{20}-(C_2H_4O)_{20}-C_4H_9]\}^+\ Cl^-$,

$-CH_2-\{N[-CH_2-CH(OH)-CH_2-NMe_3][-CH_2-CH_2-C(O)-O-(C_2H_4O)_5-H]\}^+\ Cl^-$,

$-CH_2-\{N[-CH_2-CH(OH)-CH_2-NMe_3][-CH_2-CH_2-C(O)-O-(C_3H_6O)_{16}-CH_3]\}^+\ Cl^-$,

$-CH_2-\{N[-CH_2-CH(OH)-CH_2-NMe_3][-CH_2-CH_2-C(O)-O-(C_3H_6O)_{20}-(C_2H_4O)_{20}-C(O)-CH=CH_2]\}^+ Cl^-$,

$-(CH_2)_3-\{N[-CH_2-CH(OH)-CH_2-NMe_3][-CH_2-CH(OH)-CH_2-O-(C_2H_4O)_5-CH_3]\}^+ Cl^-$,

$-(CH_2)_3-\{N[-CH_2-CH(OH)-CH_2-NMe_3][-CH_2-CH(OH)-CH_2-O-(C_3H_6O)_{20}-C_3H_5O]\}^+ Cl^-$,

$-(CH_2)_3-\{N[-CH_2-CH(OH)-CH_2-NMe_3][-CH_2-CH(OH)-CH_2-O-(C_2H_4O)_6-CH_2CH(OH)CH_2OH]\}^+ Cl^-$,

$-(CH_2)_3-\{N[-CH_2-CH(OH)-CH_2-NMe_3][-CH_2-CH_2-C(O)-O-(C_2H_4O)_{25}-C(O)CH_3]\}^+ Cl^-$,

$-(CH_2)_3-\{N[-CH_2-CH(OH)-CH_2-NMe_3][-CH_2-CH_2-C(O)-O-(C_3H_6O)_{16}-CH_3]\}^+ Cl^-$,

$-(CH_2)_3-\{N [-CH_2-CH(OH)-CH_2-NMe_3][-CH_2-CH_2-C(O)-O-(C_3H_6O)_{20}-(C_2H_4O)_{20}-C(O)-CH_2CH_2-NEt_2]\}^+ Cl^-$,

$-(CH_2)_3-NH-(CH_2)_2-\{N[-CH_2-CH(OH)-CH_2-NMe_3][-CH_2-CH(OH)-CH_2-O-(C_2H_4O)_5-C_4H_9]\}^+ Cl^-$,

$-(CH_2)_3-NH-(CH_2)_2-\{N [-CH_2-CH(OH) -CH_2-NMe_3][-CH_2-CH(OH) -CH_2-O-(C_2H_4O)_{22}-(C_3H_6O)_{19}-C_4H_9]\}^+ Cl^-$,

$-(CH_2)_3-\{N[-CH_2-CH(OH)-CH_2-NMe_3]-(CH_2)_2-N[-CH_2-CH(OH)-CH_2-O-(C_2H_4O)_{11}-C_4H_9]H\}^+ Cl^-$,

$-(CH_2)_3-N[-CH_2-CH(OH)-CH_2-O-(C_2H_4O)_{20}-C_4H_9]-(CH_2)_2-\{NH[-CH_2-CH(OH)-CH_2-NMe_3]\}^+ Cl^-$,

$-(CH_2)_3-NH-(CH_2)_2-\{N[-CH_2-CH(OH)-CH_2-NMe_3][-CH_2-CH_2-C(O)-O-(C_3H_6O)_{16}-CH_3]\}^+ Cl^-$,

$-(CH_2)_3-NH-(CH_2)_2-\{N[-CH_2-CH(OH)-CH_2-NMe_3][-CH_2-CH(OH)-CH_2-O-(C_3H_6O)_{24}-H]\}^+ Cl^-$,

$- (CH_2)_3-\{N[-CH_2-CH(OH) -CH_2-NMe_3]-(CH_2)_2-N[-CH_2-CH_2-C(O)-O-(C_2H_4O)_{35}-C(O)-CH=CH_2]H\}^+ Cl^-$,

$-(CH_2)_3-N[-CH_2-CH(OH)-CH_2-O-(C_2H_4O)_4-C_4H_9]-(CH_2)_2-\{N[-CH_2-CH(OH)-CH_2-NMe_3][-CH_2-CH(OH)-CH_2-O-(C_2H_4O)_{22}-(C_3H_6O)_{19}-C_4H_9]\}^+ Cl^-$,

$-(CH_2)_3-\{N[-CH_2-CH(OH)-CH_2-NMe_3]-(CH_2)_2-N[-CH_2-CH(OH)-CH_2-NMe_3][-CH_2-CH(OH)-CH_2-O-(C_2H_4O)_{24}-CH_3]\}^{2+} 2Cl^-$,

$-(CH_2)_3-\{N[-CH_2-CH(OH)-CH_2-NMe_3][-CH_2-CH(OH)-CH_2-(C_2H_4O)_5-C_4H_9]-(CH_2)_2-N[-CH_2-CH(OH)-CH_2-NMe_3][-CH_2-CH_2-C(O)-O-(C_2H_4O)_{15}-(C_3H_6O)_5-CH_3\}^{3+} 3Cl^-$,

$-(CH_2)_3-\{N[-CH_2-CH(OH)-CH_2-NMe_3]-(CH_2)_2-N[-CH_2-CH(OH)-CH_2-NMe_3][-CH_2-CH(OH)-CH_2-O-(C_2H_4O)_{11}-C_4H_9][-CH_2-CH_2-C(O)-0- (C_2H_4O)_{16}-CH_3]\}^{3+} 3Cl^-$,

$-(CH_2)_3-\{N[-CH_2-CH(OH)-CH_2-NMe_3]-(CH_2)_2-N[-CH_2-CH(OH)-CH_2-NMe_3]_2[-CH_2-CH(OH)-CH_2-O-(C_2H_4O)_{22}-(C_3H_6O)_{19}-C_4H_9]\}^{4+} 4Cl^-$,

$- (CH_2)_3-\{N [-CH_2-CH_2-C(O)-O-(C_2H_4O)_{35}-CH_3]-(CH_2)_2-N[-CH_2-CH(OH) -CH_2-NMe_3]_2[-CH_2-CH(OH) -CH_2-(C_2H_4O)_4-C_4H_9]\}^{4+} 4Cl^-$.

Beispiele für Reste **A** sind Wasserstoffatom, die -OH-Gruppe sowie die für $\mathbf{R^6}$ und $\mathbf{R^7}$ genannten Reste.
Beispiele für Reste **B** sind
$-SiMe_2-O-SiMe_2-$,
$- (SiMe_2O)_{13}-SiMe_2-$,
$- (SiMe_2O)_{55}-SiMe_2-$,
$- (SiMe_2O)_{105}-SiMe_2-$,
$-(SiMe_2O)_{200}-SiMe_2-$,
$-SiMe_2O-SiMeR^7O-SiMe_2O-$,
$- (SiMe_2O)_{70}(SiMeR^7O)_1-SiMe_2-$,
$-(SiMe_2O)_{105}(SiMeR^7O)_{20}-SiMe_2-$,
$- (SiMe_2O)_{195}(SiMeR^7O)_{15}-SiMe_2-$,
$- (SiMe_2O)_{230}(SiMeR^7O)_6-SiMe_2-$,

$-(SiMe_2O)_{108}(SiMeR^7O)_2-SiMe_2-$,
$-(SiMe_2O)_{139}(SiMeR^7O)_1-SiMe_2-$,
$-(SiMe_2O)_9-SiMe_3$,
$-(SiMe_2O)_{59}-SiMe_3$,
$-(SiMe_2O)_{99}-SiMe_3$,
$-(SiMe_2O)_{13}-SiMe_2-OCH_3$,
$-(SiMe_2O)_{14}-SiMe_2-OC_2H_4$,
$-(SiMe_2O)_{44}-SiMe_2-OC_3H_7$,
$-(SiMe_2O)_{22}-SiMe_2-OC_4H_9$,
$-(SiMe_2O)_{35}-SiMe_2-OC_4H_9$.

Beispiele für Reste **D** sind
$-\{CH_2-N[-CH_2-CH(OH)-CH_2-NMe_3]-J-N[-CH_2-CH(OH)-CH_2-NMe_3]-CH_2\}-^{2+}\ 2Cl^-$,
$-\{CH_2-NH-J-N[-CH_2-CH(OH)-CH_2-NMe_3]-CH_2\}-^{+}\ Cl^-$,
$-\{CH_2-N[-CH_2-CH(OH)-CH_2-NMe_3]-J-NF-CH_2\}-^{+}\ Cl^-$,
$-\{CH_2-NH-J-NF-CH_2\}-$,
$-\{CH_2-NF-J-NF-CH_2\}-$,
$-\{(CH_2)_3-N[-CH_2-CH(OH)-CH_2-NMe_3]-J-N[-CH_2-CH(OH)-CH_2-NMe_3]-(CH_2)_3\}-^{2+}\ 2Cl^-$,
$-\{(CH_2)_3-NH-J-N[-CH_2-CH(OH)-CH_2-NMe_3]-(CH_2)_3\}-^{+}\ Cl^-$,
$-\{(CH_2)_3-N[-CH_2-CH(OH)-CH_2-NMe_3]-J-NF-(CH_2)_3\}-^{+}\ Cl^-$,
$-\{(CH_2)_3-NH-J-NF-(CH_2)_3\}-$,
$-\{(CH_2)_3-NF-J-NF-(CH_2)_3\}-$,
$-\{CH_2-N[-CH_2-CH(OH)-CH_2-NMe_3]-J-N[-CH_2-CH(OH)-CH_2-NMe_3]-(CH_2)_3\}-^{2+}\ 2Cl^-$,
$-\{CH_2-N[-CH_2-CH(OH)-CH_2-NMe_3]-J-NH-(CH_2)_3\}-^{2+}\ 2Cl^-$,
$-\{CH_2-NH-J-N[-CH_2-CH(OH)-CH_2-NMe_3]-(CH_2)_3\}-^{2+}\ 2Cl^-$,
$-\{CH_2-N[-CH_2-CH(OH)-CH_2-NMe_3]-J-NF-(CH_2)_3\}-^{2+}\ 2Cl^-$,
$-\{CH_2-NF-J-N[-CH_2-CH(OH)-CH_2-NMe_3]-(CH_2)_3\}-^{2+}\ 2Cl^-$,
$-\{CH_2-NF-J-NF-(CH_2)_3\}-$,
$-\{CH_2-NF-J-NH-(CH_2)_3\}-$,
$-\{CH_2-NH-J-NF-(CH_2)_3\}-$,
wobei Me für Methylrest steht und sich durch Permutation der genannten Beispiele mit den Resten **F** und **J** eine Vielzahl weiterer Beispielstrukturen ergeben.

Beispiele für Einheiten **E** sind
$-CH_2-CH(OH)-CH_2-NMe_3^+\ Cl^-$, $-CH_2-CH(OH)-CH_2-NMe_2C_{12}H_{25}^+\ Cl^-$ und $-CH_2-CH(OH)-CH_2-NMe_2C_{18}H_{37}^+\ Cl^-$.

Beispiele für Einheiten **F** sind
$-CH_2-CH_2-C(O)-O-(C_2H_4O)_6-H$,
$-CH_2-CH_2-C(O)-O-(C_2H_4O)_6-CH_3$,
$-CH_2-CH_2-C(O)-O-(C_2H_4O)_6-C(O)-CH=CH_2$,
$-CH_2-CH_2-C(O)-O-(C_2H_4O)_6-C(O)-CH_2-CH_2-NEt_2$,
$-CH_2-CH_2-C(O)-O-(C_2H_4O)_6-C(O)-CH_2-CH_2-N(CH_2CH_2OH)_2$,
$-CH_2-CH_2-C(O)-O-(C_2H_4O)_6-C(O)CH_3$,
$-CH_2-CH_2-C(O)-O-(C_2H_4O)_{14}-H$,
$-CH_2-CH_2-C(O)-O-(C_2H_4O)_{14}-CH_3$,
$-CH_2-CH_2-C(O)-O-(C_2H_4O)_{14}-C(O)-CH=CH_2$,
$-CH_2-CH_2-C(O)-O-(C_2H_4O)_{14}-C(O)-CH_2-CH_2-NEt_2$,
$-CH_2-CH_2-C(O)-O-(C_2H_4O)_{14}-C(O)-CH_2-CH_2-N(CH_2CH_2OH)_2$,
$-CH_2-CH_2-C(O)-O-(C_2H_4O)_{14}-C(O)CH_3$,
$-CH_2-CH_2-C(O)-O-(C_2H_4O)_{35}-H$,
$-CH_2-CH_2-C(O)-O-(C_2H_4O)_{35}-CH_3$,
$-CH_2-CH_2-C(O)-O-(C_2H_4O)_{35}-C(O)-CH=CH_2$,
$-CH_2-CH_2-C(O)-O-(C_2H_4O)_{35}-C(O)-CH_2-CH_2-NEt_2$,
$-CH_2-CH_2-C(O)-O-(C_2H_4O)_{35}-C(O)-CH_2-CH_2-N(CH_2CH_2OH)_2$,
$-CH_2-CH_2-C(O)-O-(C_2H_4O)_{35}-C(O)CH_3$,
$-CH_2-CH_2-C(O)-O-(C_3H_6O)_6-H$,
$-CH_2-CH_2-C(O)-O-(C_3H_6O)_6-CH_3$,
$-CH_2-CH_2-C(O)-O-(C_3H_6O)_6-C(O)-CH=CH_2$,
$-CH_2-CH_2-C(O)-O-(C_3H_6O)_6-C(O)-CH_2-CH_2-NEt_2$,
$-CH_2-CH_2-C(O)-O-(C_3H_6O)_6-C(O)-CH_2-CH_2-N(CH_2CH_2OH)_2$,
$-CH_2-CH_2-C(O)-O-(C_3H_6O)_6-C(O)CH_3$,

$-CH_2-CH_2-C(O)-O-(C_3H_6O)_{12}-H$,
$-CH_2-CH_2-C(O)-O-(C_3H_6O)_{12}-CH_3$,
$-CH_2-CH_2-C(O)-O-(C_3H_6O)_{12}-C(O)-CH=CH_2$,
$-CH_2-CH_2-C(O)-O-(C_3H_6O)_{12}-C(O)-CH_2-CH_2-NEt_2$,
$-CH_2-CH_2-C(O)-O-(C_3H_6O)_{12}-C(O)-CH_2-CH_2-N(CH_2CH_2OH)_2$,
$-CH_2-CH_2-C(O)-O-(C_3H_6O)_{12}-C(O)CH_3$,
$-CH_2-CH_2-C(O)-O-(C_3H_6O)_{35}-H$,
$-CH_2-CH_2-C(O)-O-(C_3H_6O)_{35}-CH_3$,
$-CH_2-CH_2-C(O)-O-(C_3H_6O)_{35}-C(O)-CH=CH_2$,
$-CH_2-CH_2-C(O) -O- (C_3H_6O)_{35}-C (O) -CH_2-CH_2-NEt_2$,
$-CH_2-CH_2-C(O)-O-(C_3H_6O)_{35}-C(O)-CH_2-CH_2-N(CH_2CH_2OH)_2$,
$-CH_2-CH_2-C(O)-O-(C_3H_6O)_{35}-C(O)CH_3$,
$-CH_2-CH_2-C(O)-O-(C_3H_6O)_5-(C_2H_4O)_5-H$,
$-CH_2-CH_2-C(O)-O-(C_3H_6O)_5-(C_2H_4O)_5-CH_3$,
$-CH_2-CH_2-C(O)-O-(C_3H_6O)_5-(C_2H_4O)_5C(O)-CH=CH_2$,
$-CH_2-CH_2-C(O)-O-(C_3H_6O)_5-(C_2H_4O)_5C(O)-CH_2-CH_2-NEt_2$,
$-CH_2-CH_2-C(O)-O-(C_3H_6O)_5-(C_2H_4O)_5C(O)-CH_2-CH_2-N(CH_2CH_2OH)_2$,
$-CH_2-CH_2-C(O)-O-(C_3H_6O)_5-(C_2H_4O)_5-C(O)CH_3$,
$-CH_2-CH_2-C(O)-O-(C_3H_6O)_{10}-(C_2H_4O)_{10}-H$,
$-CH_2-CH_2-C(O)-O-(C_3H_6O)_{10}-(C_2H_4O)_{10}-CH_3$,
$-CH_2-CH_2-C(O)-O-(C_3H_6O)_{10}-(C_2H_4O)_{10}C(O)-CH=CH_2$,
$-CH_2-CH_2-C(O)-O-(C_3H_6O)_{10}-(C_2H_4O)_{10}C(O)-CH_2-CH_2-NEt_2$,
$-CH_2-CH_2-C(O)-O-(C_3H_6O)_{10}-(C_2H_4O)_{10}C(O)-CH_2-CH_2-N(CH_2CH_2OH)_2$,
$-CH_2-CH_2-C(O)-O-(C_3H_6O)_{10}-(C_2H_4O)_{10}-C(O)CH_3$,
$-CH_2-CH(OH)-CH_2-O-(C_2H_4O)_4-C_4H_9$
$-CH_2-CH(OH)-CH_2-O-(C_2H_4O)_{11}-C_4H_9$,
$-CH_2-CH(OH)-CH_2-O-(C_2H_4O)_{20}-C_4H_9$,
$-CH_2-CH(OH)-CH_2-O-(C_2H_4O)_{22}(C_3H_6O)_{19}-C_4H_9$,
$-CH_2-CH(OH)-CH_2-O-(C_2H_4O)_4-C_3H_5O$,
$-CH_2-CH(OH)-CH_2-O-(C_2H_4O)_{11}-C_3H_5O$,
$-CH_2-CH(OH)-CH_2-O-(C_2H_4O)_{20}-C_3H_5O$,
$-CH_2-CH(OH) -CH_2-O-(C_2H_4O)_{22}(C_3H_6O)_{19}-C_3H_5O$,
$-CH_2-CH(OH) -CH_2-O-(C_2H_4O)_4-CH_2-CH(OH)-CH_2-OH$,
$-CH_2-CH(OH)-CH_2-O-(C_2H_4O)_{11}-CH_2-CH(OH)-CH_2-OH$,
$-CH_2-CH(OH)-CH_2-O-(C_2H_4O)_{20}-CH_2-CH(OH)-CH_2-OH$,
$-CH_2-CH(OH) -CH_2-O-(C_2H_4O)_{22}(C_3H_6O)_{19}-CH_2-CH(OH)-CH_2-OH$,
$-CH_2-CH(OH)-CH_2-O-(C_2H_4O)_4-CH_2-CH(OH)-CH_2-NEt_2$,
$-CH_2-CH(OH)-CH_2-O-(C_2H_4O)_{11}-CH_2-CH(OH)-CH_2-NEt_2$,
$-CH_2-CH(OH)-CH_2-O-(C_2H_4O)_{20}-CH_2-CH(OH)-CH_2-NEt_2$,
$-CH_2-CH(OH)-CH_2-O-(C_2H_4O)_{22}(C_3H_6O)_{19}-CH_2-CH(OH)-CH_2-NEt_2$,
$-CH_2-CH(OH)-CH_2-O-(C_2H_4O)_4-CH_2-CH(OH)-CH_2-N(CH_2CH_2OH)_2$,
$-CH_2-CH(OH)-CH_2-O-(C_2H_4O)_{11}-CH_2-CH(OH)-CH_2-N(CH_2CH_2OH)_2$,
$-CH_2-CH(OH)-CH_2-O-(C_2H_4O)_{20}-CH_2-CH(OH)-CH_2-N(CH_2CH_2OH)_2$,
$-CH_2-CH(OH)-CH_2-O-(C_2H_4O)_{22}(C_3H_6O)_{19}-CH_2-CH(OH)-CH_2-N(CH_2CH_2OH)_2$,
wobei Me für Methylrest und Et für Ethylrest steht.
Beispiele für Reste J sind
$-CH_2-CH_2-C(O)-O-(C_2H_4O)_6-C(O)-CH_2-CH_2-$,
$-CH_2-CH_2-C(O)-O-(C_2H_4O)_{14}-C(O)-CH_2-CH_2-$,
$-CH_2-CH_2-C(O)-O-(C_2H_4O)_{35}-C(O)-CH_2-CH_2-$,
$-CH_2-CH_2-C(O)-O-(C_3H_6O)_6-C(O)-CH_2-CH_2-$,
$-CH_2-CH_2-C(O)-O-(C_3H_6O)_{12}-C(O)-CH_2-CH_2-$,
$-CH_2-CH_2-C(O)-O-(C_3H_6O)_{35}-C(O)-CH_2-CH_2-$,
$-CH_2-CH_2-C(O)-O-(C_3H_6O)_5-(C_2H_4O)_5-C(O)-CH_2-CH_2-$,
$-CH_2-CH_2-C(O)-O-(C_3H_6O)_{10}-(C_2H_4O)_{10}-C(O)-CH_2-CH_2-$,
$-CH_2-CH_2-C(O)-O-(C_3H_6O)_5-(C_2H_4O)_{15}-C(O)-CH_2-CH_2-$,
$-CH_2-CH_2-C(O)-O-(C_3H_6O)_{20}-(C_2H_4O)_{20}-C(O)-CH_2-CH_2-$,
$-CH_2-CH_2-C(O)-NH- (C_3H_6O)_3-C_3H_6-NH-C(O)-CH_2-CH_2-$,
$-CH_2-CH_2-C(O)-NH- (C_3H_6O)_6-C_3H_6-NH-C(O)-CH_2-CH_2-$,

$-CH_2-CH_2-C(O)-NH-(C_3H_6O)_{33}-C_3H_6-NH-C(O)-CH_2-CH_2-$,
$-CH_2-CH_2-C(O)-NH-(C_3H_6O)_{68}-C_3H_6-NH-C(O)-CH_2-CH_2-$,
$-CH_2-CH_2-C(O)-NH-C_3H_6O-(C_2H_4O)_2-C_3H_6-NH-C(O)-CH_2-CH_2-$,
$-CH_2-CH_2-C(O)-NH-(C_3H_6O)_2-(C_2H_4O)_9-C_3H_6O-C_3H_6-NH-C(O)-CH_2-CH_2-$,
$-CH_2-CH_2-C(O)-NH-(C_3H_6O)_3-(C_2H_4O)_{39}-(C_3H_6O)_2-C_3H_6-NH-C(O)-CH_2-CH_2-$,
$-CH_2-CH(OH)-CH_2-O-(C_2H_4O)_4-CH_2-CH(OH)-CH_2-$,
$-CH_2-CH(OH)-CH_2-O-(C_2H_4O)_{11}-CH_2-CH(OH)-CH_2-$,
$-CH_2-CH(OH)-CH_2-O-(C_2H_4O)_{20}-CH_2-CH(OH)-CH_2-$,
$-CH_2-CH(OH)-CH_2-O-(C_2H_4O)_{22}(C_3H_6O)_{19}-CH_2-CH(OH)-CH_2-$.

Beispiele erfindungsgemäß geeigneter Anionen $X^-$ sind das Chloridion, Bromidion, Hydrogensulfation, Acetation, Sulfation und organische Sulfonationen wie Trifluormethansulfonation oder p-Toluolsulfonation.

Gegenstand der Erfindung ist weiterhin ein Verfahren zur Herstellung von Copolymeren (Q) der vorstehenden allgemeinen Formel (I), bei dem Organosiliciumverbindungen oder Mischungen davon, die ausgewählt werden aus Verbindungen der allgemeinen Formeln (XI), (XII), (XIII), (XIV) und (XV)

$$H-NR^1-(R-NHR^1)_g-R-(SiR^6{}_2O)_1-SiR^6{}_2-R(-NHR^1-R)_g-NR^1-H \quad (XI),$$

$$H-NR^1-(R-NHR^1)_g-R-(SiR^6{}_2O)_1-\{SiR^6[-R(-NHR^1-R)_g-NHR^1]-O\}_m-R(-NHR^1-R)_g-NR^1-H \quad (XII),$$

$$R^6_3SiO-(SiR^6{}_2O)_1-\{SiR^6[-R(-NHR^1-R)_g-NHR^1]-O\}_m-SiR^6{}_3 \quad (XIII),$$

$$MeO-SiR^6{}_2O-(SiR^6{}_2O)_1-\{SiR^6[-R(-NHR^1-R)_g-NHR^1]-O\}_m-SiR^6{}_2-OMe \quad (XIV),$$

$$H-NR^1-(R-NHR^1)_g-R-(SiR^6{}_2O)_1-SiR^6{}_2-R^8 \quad (XV),$$

mit Polyethern, die ausgewählt werden aus Verbindungen der allgemeinen Formeln (XVI) und (XVII)

$$CH_2=CH-C(O)-G-(R^2-G)_h-[CH_2CH_2O]_i-[C_3H_6O]_j-[(CH_2)_4O]_k-(R^2-G)_h-R^5 \quad (XVI),$$

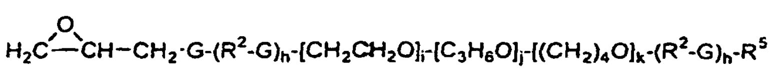

(XVII)

und organischen Verbindungen, die ausgewählt werden aus Verbindungen der allgemeinen Formeln (XVIII) und (XIX)

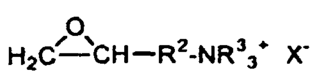

(XVIII)

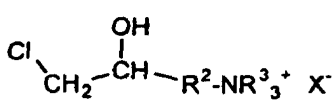

(XIX)

umgesetzt werden, wobei **Me** Methylrest bedeutet und **$R^1$, $R^2$, $R^3$, $R^5$, $R^6$, G, X, g, h, i, j, k, l,** sowie **m** die vorstehend dafür angegebenen Bedeutungen aufweisen.

Das Verfahren kann in Substanz, Lösung oder Emulsion sowohl stufenweise als auch als Eintopfverfahren

durchgeführt werden. Die eingesetzten Organosiliciumverbindungen sind bekannt und käuflich erhältlich.

Die Einführung der kationischen Quatgruppe erfolgt beim vorliegenden Verfahren auf andere Art und Weise als nach dem Stand der Technik üblich. Beim vorliegenden Verfahren wird ein wässriges Reagenz verwendet. Wässrige Systeme sind per se inkompatibel mit hydrophoben Siloxanen, die Modifikation von Silicon-Ölen gelingt üblicherweise mit wässrigen Reagenzien daher nur unter Schwierigkeiten und mit schlechter Ausbeute. Beim vorliegenden Verfahren werden gute Ausbeuten erreicht.

Beispiele für eingesetzte Organosiliciumverbindungen der allgemeinen Formel (XI) sind
$H_2N-CH_2-SiMe_2O-SiMe_2-CH_2-NH_2$,
$H_2N-CH_2-SiMe_2O-(SiMe_2O)_6-SiMe_2-CH_2-NH_2$,
$H_2N-CH_2-SiMe_2O-(SiMe_2O)_{11}-SiMe_2-CH_2-NH_2$,
$H_2N-CH_2-SiMe_2O-(SiMe_2O)_{98}-SiMe_2-CH_2-NH_2$,
$H_2N-CH_2-SiMe_2O-(SiMe_2O)_{150}-SiMe_2-CH_2-NH_2$,
$H_2N-(CH_2)_3-SiMe_2O-SiMe_2-(CH_2)_3-NH_2$,
$H_2N-(CH_2)_3-SiMe_2O-(SiMe_2O)_6-SiMe_2-(CH_2)_3-NH_2$,
$H_2N-(CH_2)_3-SiMe_2O-(SiMe_2O)_{11}-SiMe_2-(CH_2)_3-NH_2$,
$H_2N-(CH_2)_3-SiMe_2O-(SiMe_2O)_{98}-SiMe_2-(CH_2)_3-NH_2$,
$H_2N-(CH_2)_3-SiMe_2O-(SiMe_2O)_{200}-SiMe_2-(CH_2)_3-NH_2$,
$H_2N-(CH_2)_2-NH-(CH_2)_3-SiMe_2O-SiMe_2-(CH_2)_3-NH-(CH_2)_2-NH_2$,
$H_2N-(CH_2)_2-NH-(CH_2)_3-SiMe_2O-(SiMe_2O)_6-SiMe_2-(CH_2)_3-NH-(CH_2)_2-NH_2$,
$H_2N-(CH_2)_2-NH-(CH_2)_3-SiMe_2O-(SiMe_2O)_{11}-SiMe_2-(CH_2)_3-NH-(CH_2)_2-NH_2$,
$H_2N-(CH_2)_2-NH-(CH_2)_3-SiMe_2O-(SiMe_2O)_{55}-SiMe_2-(CH_2)_3-NH-(CH_2)_2-NH_2$.

Beispiele für eingesetzte Organosiliciumverbindungen der allgemeinen Formel (XII) sind
$H_2N-(CH_2)_3-SiMe_2O-\{SiMe[(CH_2)_3-NH_2]O\}-SiMe_2-(CH_2)_3-NH_2$,
$H_2N-(CH_2)_3-SiMe_2O-(SiMe_2O)_{25}-\{SiMe[(CH_2)_3-NH_2]O\}_5-SiMe_2-(CH_2)_3-NH_2$,
$H_2N-(CH_2)_3-SiMe_2O-(SiMe_2O)_{50}-\{SiMe[(CH_2)_3-NH_2]O\}_3-SiMe_2-(CH_2)_3-NH_2$,
$H_2N-(CH_2)_3-SiMe_2O-(SiMe_2O)_{70}-\{SiMe[(CH_2)_3-NH_2]O\}_1-SiMe_2-(CH_2)_3-NH_2$.

Beispiele für eingesetzte Organosiliciumverbindungen der allgemeinen Formel (XIII) sind
$Me_3SiO-(SiMe_2O)_{25}-\{SiMe[(CH_2)_3-NH_2]O\}SiMe_3$,
$Me_3SiO-(SiMe_2O)_{45}-\{SiMe[(CH_2)_3-NH_2]O\}_5SiMe_3$,
$Me_3SiO-(SiMe_2O)_{100}-\{SiMe[(CH_2)_3-NH_2]O\}_5SiMe_3$,
$Me_3SiO-(SiMe_2O)_{130}-\{SiMe[(CH_2)_3-NH_2]O\}_1SiMe_3$,
$Me_3SiO-(SiMe_2O)_{105}\{SiMe[(CH_2)_3-NH-(CH_2)_2-NH_2]O\}_{20}-SiMe_3$,
$Me_3SiO-(SiMe_2O)_{195}(SiMe[(CH_2)_3-NH-(CH_2)_2-NH_2]O)_{15}-SiMe_3$,
$Me_3SiO-(SiMe_2O)_{230}(SiMe[(CH_2)_3-NH-(CH_2)_2-NH_2]O)_6-SiMe_3$,
$Me_3SiO-(SiMe_2O)_{108}(SiMe[(CH_2)_3-NH-(CH_2)_2-NH_2]O)_2-SiMe_3$,
$Me_3SiO-(SiMe_2O)_{139}(SiMe[(CH_2)_3-NH-(CH_2)_2-NH_2]O)_1-SiMe_3$.

Beispiele für eingesetzte Organosiliciumverbindungen der allgemeinen Formel (XIV) sind
$Me_3SiO-(SiMe_2O)_{25}-\{SiMe[(CH_2)_3-NH_2]O\}_5SiMe_3$,
$Me_3SiO-(SiMe_2O)_{45}-\{SiMe[(CH_2)_3-NH_2]O\}_5SiMe_3$,
$Me_3SiO-(SiMe_2O)_{100}-\{SiMe[(CH_2)_3-NH_2]O\}_5SiMe_3$,
$Me_3SiO-(SiMe_2O)_{130}-\{SiMe[(CH_2)_3-NH_2]O\}_1SiMe_3$,
$MeOSiMe_2O-(SiMe_2O)_{40}\{SiMe[(CH_2)_3-NH-(CH_2)_2-NH_2]O\}_1-SiMe_2OMe$,
$MeOSiMe_2O-(SiMe_2O)_{70}\{SiMe[(CH_2)_3-NH-(CH_2)_2-NH_2]O\}_1-SiMe_2OMe$,
$MeOSiMe_2O-(SiMe_2O)_{200}\{SiMe[(CH_2)_3-NH-(CH_2)_2-NH_2]O\}_1-SiMe_2OMe$.

Beispiele für eingesetzte Organosiliciumverbindungen der allgemeinen Formel (XV) sind
$H_2N-CH_2-(SiMe_2O)_{13}-SiMe_2-OCH_3$,
$H_2N-CH_2-(SiMe_2O)_{14}-SiMe_2-OC_2H_4$,
$H_2N-CH_2-(SiMe_2O)_{44}-SiMe_2-OC_3H_7$,
$H_2N-CH_2-(SiMe_2O)_{22}-SiMe_2-OC_4H_9$,
$H_2N-CH_2-(SiMe_2O)_{35}-SiMe_2-OC_4H_9$.
$H_2N-(CH_2)_3-(SiMe_2O)_9-SiMe_3$,
$H_2N-(CH_2)_3-(SiMe_2O)_{59}-SiMe_3$,
$H_2N-(CH_2)_3-(SiMe_2O)_{99}-SiMe_3$,
$H_2N-(CH_2)_3-(SiMe_2O)_{13}-SiMe_2-OCH_3$,
$H_2N-(CH_2)_3-(SiMe_2O)_{14}-SiMe_2-OC_2H_4$,
$H_2N-(CH_2)_3-(SiMe_2O)_{44}-SiMe_2-OC_3H_7$,
$H_2N-(CH_2)_3-(SiMe_2O)_{22}-SiMe_2-OC_4H_9$,
$H_2N-(CH_2)_3-(SiMe_2O)_{35}-SiMe_2-OC_4H_9$.

$H_2N$- $(CH_2)$ $_2$-NH- $(CH_2)$ $_3$- $(SiMe_2O)_7$-$SiMe_3$,
$H_2N$-$(CH_2)_2$-NH-$(CH_2)_3$-$(SiMe_2O)_{12}$-$SiMe_3$.
wobei Organosiliciumverbindungen der allgemeinen Formeln (XI), (XII), (XIII) und (XV) bevorzugt sind, insbesondere Organosiliciumverbindungen der allgemeinen Formeln (XI), (XIII) und (XV).

Beispiele für eingesetzte, ebenfalls käuflich erhältliche (z. B. Fa. Cray Valley, Siber Hegner AG, NOF Corp.) oder nach dem Fachmann bekannten Methoden herstellbare Polyether der allgemeinen Formeln (XVI) und (XVII) sind
$CH_2$=CH-C (O) -O- $(C_2H_4O)_6$-H,
$CH_2$=CH-C(O)-O-$(C_2H_4O)_{14}$-H,
$CH_2$=CH-C(O)-O-$(C_2H_4O)_{35}$-H,
$CH_2$=CH-C(O)-O-$(C_3H_6O)_6$-H,
$CH_2$=CH-C(O)-0- $(C_3H_6O)_{12}$-H,
$CH_2$=CH-C(O)-0- $(C_3H_6O)_{35}$-H,
$CH_2$=CH-C(O)-0- $(C_2H_4O)_3$-$(C_3H_6O)_3$-H,
$CH_2$=CH-C(O)-O-$(C_2H_4O)_5$-$(C_3H_6O)_5$-H,
$CH_2$=CH-C(O)-O-$(C_2H_4O)_{10}$-$(C_3H_6O)_{10}$-H,
$CH_2$=CH-C(O)-O-$(C_2H_4O)_6$-$CH_3$,
$CH_2$=CH-C (O) -O- $(C_2H_4O)$ $_{16}$-$CH_3$,
$CH_2$=CH-C(O)-O-$(C_2H_4O)_{24}$-$CH_3$,
$CH_2$=CH-C(O)-O-$(C_2H_4O)_{25}$-$CH_3$,
$CH_2$=CH-C(O)-0- $(C_3H_6O)_6$-$CH_3$,
$CH_2$=CH-C (O) -O- $(C_3H_6O)_{12}$-$CH_3$,
$CH_2$=CH-C(O)-O-$(C_3H_6O)_{35}$-$CH_3$,
$CH_2$=CH-C(O)-O-$(C_2H_4O)_3$-$(C_3H_6O)_3$-$CH_3$,
$CH_2$=CH-C(O)-O-$(C_2H_4O)_5$-$(C_3H_6O)_5$-$CH_3$,
$CH_2$=CH-C(O)-O-$(C_2H_4O)_{10}$-$(C_3H_6O)_{10}$-$CH_3$,
$CH_2$=CH-C (O)-O- $(C_2H_4O)_3$-C(O)-CH=$CH_2$,
$CH_2$=CH-C (O) -O- $(C_2H_4O)_6$-C(O) -CH=$CH_2$,
$CH_2$=CH-C (O)-O-$(C_2H_4O)_{14}$-C(O)-CH=$CH_2$,
$CH_2$=CH-C(O)-O-$(C_2H_4O)_{35}$-C(O)-CH=$CH_2$,
$CH_2$=CH-C(O)-O-$(C_3H_6O)_3$-C(O)-CH=$CH_2$,
$CH_2$=CH-C(O)-O-$(C_3H_6O)_6$-C(O)-CH=$CH_2$,
$CH_2$=CH-C (O) -O- $(C_3H_6O)_{12}$-C (O) -CH=$CH_2$,
$CH_2$=CH-C(O)-O-$(C_3H_6O)_{35}$-C(O)-CH=$CH_2$,
$CH_2$=CH-C(O)-O-$(C_2H_4O)_3$-$(C_3H_6O)_3$-C(O)-CH=$CH_2$,
$CH_2$=CH-C(O)-O-$(C_2H_4O)_5$-$(C_3H_6O)_5$-C(O)-CH=$CH_2$,
$CH_2$=CH-C(O)-0- $(C_2H_4O)_{10}$-$(C_3H_6O)_{10}$-C(O)-CH=$CH_2$,

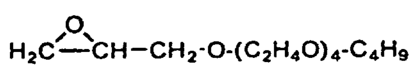

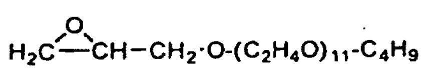

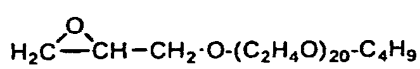

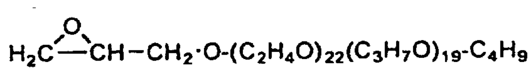

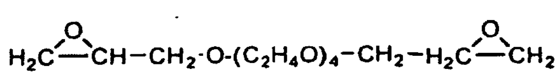

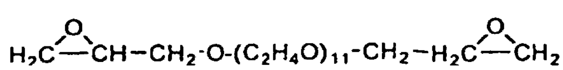

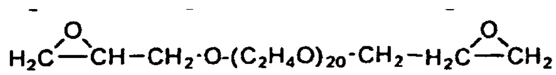

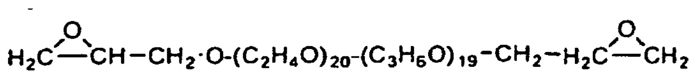

Beispiele für eingesetzte, ebenfalls käuflich erhältliche organische Verbindungen der allgemeinen Formeln (XVIII) und (XIX) (z. B. Degussa AG) sind

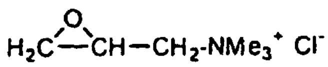

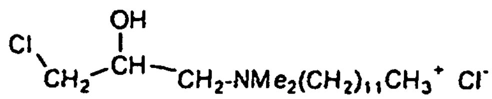

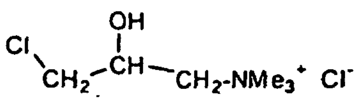

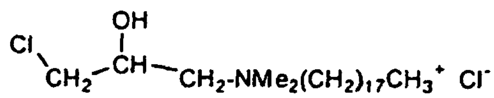

Bei dem Verfahren werden pro Mol Amingruppe der Organosiliciumverbindung der allgemeinen Formeln (XI), (XII), (XIII), (XIV) und (XV) vorzugsweise mindestens 0,001 mol, insbesondere mindestens 0,1 mol, besonders bevorzugt mindestens 0,5 und vorzugsweise höchstens 1,75 mol Polyether der allgemeinen Formeln (XVI) und (XVII), sowie vorzugsweise mindestens 0,001 mol, insbesondere mindestens 0,1 mol, besonders bevorzugt mindestens 0,5 und vorzugsweise höchstens 1,75 mol organische Verbindung der allgemeinen Formeln (XVIII) und (XIX) eingesetzt.
Kommen bei dem Verfahren difunktionelle Polyetherverbindung der allgemeinen Formeln (XVI) und (XVII) zum Einsatz und werden die Anteile Polyetherverbindung der allgemeinen Formeln (XVI) und (XVII) und der organischen Verbindung der allgemeinen Formeln (XVIII) und (XIX) so gewählt, daß in den Copolymeren der allgemeinen Formel (I) überschüssige Acrylat- und Epoxidreste auftreten, so können diese reaktiven Endgruppen nachträglich vernetzen oder gezielt vernetzt werden.
Die Produkte der allgemeinen Formel (I) mit reaktiven Acrylatund Epoxidendgruppen können gegen radikalische Vernetzung an Tageslicht durch die üblichen Radikalinhibitoren wie beispielsweise Hydrochinonmonomethylether oder Phenothiazin über mehrere Monate lagerstabil gemacht werden. Eine Stabilisierung gegen eine mögliche Nachvernetzung durch im Produkt verbleibende primäre oder sekundäre Amingruppen mit den Acrylat- oder Epoxidendgruppen kann durch Amidierung dieser Amingruppen mit organischen Säureanhydriden oder Säurechloriden, oder durch eine der eigentlichen Reaktion nachfolgende Michaelreaktion-ähnliche Umsetzung zur Absättigung der Amingruppen mit monomeren Acrylsäureesterverbindungen erfolgen. Verbleibende primäre oder sekundäre Amingruppen in den Copolymeren der allgemeinen Formel (I) können darüber hinaus auch protoniert, alkyliert oder quarternisiert werden; dies gilt auch für tertiäre Amingruppen. Bei dem Verfahren zur Herstellung der Copolymere der allgemeinen Formel (I) können aus der Literatur bekannte Verbindungen, die Michaelreaktion-ähnliche Umsetzungen und Epoxidring-Öffnungs-Reaktionen katalysieren, eingesetzt werden. Beispiele sind Brønsted-Säuren, wie Phosphorsäure, Schwefelsäure, Salzsäure, Eisessig, Propion- und Ameisensäure, oder deren wässrige Lösungen, Lewis-Säuren, wie Litiumperchlorat, Zinktetrafluoroborat, Eisen(II)chlorid und Zinn(IV)chlorid, und Brønsted-Basen, wie z.B. Natriummethylat und Alkaliamide, sowie Ammoniumchlorid, Tetraalkylammoniumbromide und Alkaliiodide. Bei dem Verfahren können organische Lösungsmittel, Wasser, oder Mischungen aus beiden, die oben genannten Polyether der allgemeinen Formeln (XVI) und (XVII) sowie das Reaktionsprodukt selbst als Lösungsmittel mitverwendet werden. Beispiele für organische Lösungsmittel sind Toluol, Xylol, THF, n-Butylacetat, Isopropanol, Butanol und Dimethoxyethan. Vorzugsweise wird mitverwendetes organisches Lösungsmittel nach der Reaktion, bzw. nach den Folgereaktionen wieder entfernt.

Wird das Verfahren in Emulsion durchgeführt so können entsprechend Emulgatoren bzw. oberflächenaktive Mittel vorhanden sein. Auch kann bei der Durchführung des Verfahrens in Emulsion das Reaktionsprodukt selbst aufgrund seiner oberflächenaktiven Eigenschaften als Emulgator oder Coemulgator verwendet werden. Die Copolymere der allgemeinen Formel (I) können mit Organopolysiloxanen, ausgewählt aus der Gruppe bestehend aus linearen, endständigen Triorganosiloxygruppen aufweisenden Organopolysiloxanen, lineare, endständige Hydroxylgruppen aufweisenden Organopolysiloxanen, cyclischen Organopolysiloxanen und Mischpolymerisaten aus Diorganosiloxan-

und Monoorganosiloxaneinheiten equilibriert werden.
Vorzugsweise werden als lineare, endständige Triorganosiloxygruppen aufweisende Organopolysiloxane solche der allgemeinen Formel (XX)

$$R^6{}_3SiO(SiR^6{}_2O)_qSiR^6{}_3 \quad (XX),$$

vorzugsweise werden als lineare, endständige Hydroxylgruppen aufweisende Organopolysiloxane solche der allgemeinen Formel (XXI)

$$HO(SiR^6{}_2O)_rH \quad (XXI),$$

vorzugsweise werden als cyclische Organopolysiloxane solche der allgemeinen Formel (XXII)

$$(R^6{}_2SiO)_s \quad (XXII),$$

und vorzugsweise werden als Mischpolymerisate solche aus Einheiten der allgemeinen Formeln (XXIII), (XXIV) und (XXV)

$$R^6{}_3SiO_{1/2},\ R^6{}_2SiO \text{ und } R^6SiO_{3/2} \quad (XXIII, XXIV, XXV),$$

eingesetzt, wobei
**q** und **r** jeweils den Wert 0 oder eine ganze Zahl im Wert von 1 bis 1500 und,
**s** eine ganze Zahl von 3 bis 12 bedeuten und $\mathbf{R^6}$ die oben dafür angegebene Bedeutung hat.

Die Mengenverhältnisse der bei der gegebenenfalls durchgeführten Equilibrierung eingesetzten Organopolysiloxane der allgemeinen Formeln (XX) bis (XXV) und der Copolymeren der allgemeinen Formel (I) werden lediglich durch den gewünschten Anteil an Polyether- und/oder Quatgruppen in den bei der gegebenenfalls durchgeführten Equilibrierung erzeugten Copolymeren und durch die gewünschte mittlere Kettenlänge der Siloxaneinheiten bestimmt.
Bei der gegebenenfalls durchgeführten Equilibrierung werden saure oder bevorzugt basische Katalysatoren, welche die Equilibrierung fördern, eingesetzt.
Beispiele für saure Katalysatoren sind vorzugsweise Schwefelsäure, Phosphorsäure, Trifluormethansulfonsäure, Phosphornitridchloride und unter den Reaktionsbedingungen feste, saure Katalysatoren, wie säureaktivierte Bleicherde, saure Zeolithe, sulfonierte Kohle und sulfoniertes Styrol-Divinylbenzol-Mischpolymerisat, wobei als saure Katalysatoren Phosphornitridchloride bevorzugt sind. Saure Katalysatoren werden vorzugsweise in Mengen von 5 bis 1000 Gewichts-ppm (= Teile je Million), insbesondere 50 bis 200 Gewichts-ppm, jeweils bezogen auf das Gesamtgewicht der eingesetzten Organosiliciumverbindungen, verwendet.
Beispiele für basische Katalysatoren sind Benzyltrimethylammoniumhydroxid, Tetramethylammoniumhydroxid, Alkalihydroxide, Erdalkalihydroxide in methanolischer Lösung, Phosphoniumhydroxide und Silanolate. Bevorzugt sind Ammoniumhydroxide, welche in Mengen von 50 bis 10000 Gewichts-ppm (= Teile je Million), insbesondere 500 bis 2000 Gewichts-ppm, jeweils bezogen auf das Gesamtgewicht der eingesetzten Organosiliciumverbindungen, verwendet werden.
Die gegebenenfalls durchgeführte Equilibrierung wird vorzugsweise bei 80 bis 150°C und beim Druck der umgebenden Atmosphäre, also etwa zwischen 900 und 1100 hPa, durchgeführt. Falls erwünscht, können aber auch höhere oder niedrigere Drücke angewendet werden.
Das Equilibrieren kann in mit Wasser nicht mischbarem Lösungsmittel, wie Toluol, durchgeführt werden. Falls Lösungsmittel eingesetzt werden, handelt es sich um Mengen von vorzugsweise in 5 bis 20 Gewichtsprozent, bezogen auf das Gesamtgewicht der jeweils eingesetzten Organosiliciumverbindungen.
Vor dem Aufarbeiten des bei dem Equilibrieren erhaltenen Gemisches kann der Katalysator unwirksam gemacht werden. Vorteile des Verfahrens zur Herstellung der Copolymeren der allgemeinen Formel (I) sind die einfache Durchführung in Form einer Eintopfsynthese und die Verwendung einfacher, kommerziell in großer Varietät erhältlicher Ausgangsstoffe.
Weiterhin besitzt das Verfahren zur Herstellung der Copolymeren den Vorteil, daß nicht nur durch die Wahl der Edukte die Eigenschaften der Copolymere in einfacher Weise variiert werden kann sondern auch in gewissen Grenzen durch die Stöchiometrie der Edukte bei ansonsten annähernd gleichen Reaktionsparametern, neben der Viskosität auch

einfach die Hydrophilie, die Aminzahl und die Art der Endgruppen der Produkte, sowie, das Verhältnis der Siloxan- zu Polyether- und/oder Quateinheiten variiert werden kann.

Die Copolymere (Q) der allgemeinen Formel (I) zeichnen sich aus durch Transparenz, geringe Verfärbung, Hydrolysebeständigkeit und Übergangsmetallfreiheit. Auf Grund ihrer Kationogenität, welche durch die Anzahl der quartären Aminogruppen im Molekül bedingt ist, haften die erfindungsgemäßen Copolymere sehr gut auf Substraten wie Textilien oder Papier und zeichnen sich durch ihre für Organosiliciumverbindungen vergleichsweise hohe Hydrophilie aus.

Die Copolymere (Q) können daher z. B. als Bestandteile von Emulsionen, in Lösung oder in Substanz der Behandlung von textilen Flächengebilden, wie z. B. Geweben, Maschenwaren oder Fliesen, der Textilfaser- und Garnpräparation und -modifikation sowie der Leder- und Papierbehandlung dienen. Durch die Ausrüstung oder Modifikation mit den entsprechenden Copolymeren (Q) können gewünschte Eigenschaften wie z.B. ein weicher, fließender Griff, verbesserte Elastizität, antistatische Eigenschaften, Reibwerte, Oberflächenglätte, Glanz, Knittererholung, Farbechtheiten, Waschbeständigkeit, Hydrophilie, Weiterreissfestigkeit, verringerte Pillingneigung, "Easy-Care" und "Soil-Release" Eigenschaften, sowie verbesserter Tragekomfort verliehen werden. Die durch die Ausrüstung mit den Copolymeren (Q) erzielten Effekte weisen, je nach Struktur des Copolymeren(Q), des Substrates und der Waschbedingungen, eine gute bis sehr gute Beständigkeit gegenüber Wasch- und Pflegeprozessen auf.

Weiterhin können durch die Ausrüstung oder Modifikation von textilen Flächengebilden, Fasern, Garnen, Papier und Leder mit den Copolymeren (Q) die industrielle Verarbeitbarkeit, wie z.B. die Verarbeitungs- und Produktionsgeschwindigkeit, sowie die Qualität der Materialien verbessert werden.

Die textilen Flächengebilde Fasern und Garne können gefertigt sein aus Mineralfasern, wie Glasfasern oder Silikatfasern, Naturfasern wie z.B. Wolle, Seide oder Baumwolle, Kunstfasern, wie z.B. Polyester- oder Polyamidfasern, Cellusosefasern, Mischpolymerfasern oder Metallfasern. Filamentfasern oder Stapelfasern aus den genannten Substraten können ebenfalls eingesetzt werden. Des weiteren können auch Flächengebilde aus Fasermischungen, wie z.B. Baumwolle/Polyester, Papier sowie natürliche Flächengebilde, wie Leder, verwendet werden.

Die Beschichtung oder Ausrüstung kann im Rakelverfahren, Tauch (Quetsch) (Dip)-Verfahren, Extrusionsverfahren, Spritz-Beflockungs- oder Sprühverfahren, Foulard-, Auszieh- oder Tauch-Schleuder-Verfahren aufgebracht werden. Auch sind alle Arten von Rollenbeschichtungen, wie Gravurwalzen, Pflatschen oder Auftrag über Mehrwalzensysteme, sowie Druck, z.B. (Rotary) Siebdruck, möglich. Des weiteren kann die Ausrüstung oder Beschichtung durch Schaumauftrag und nachträgliches Kalandrieren, mit einem Kalander oder Schmelzkalander erfolgen.

Des weiteren können die Copolymeren (Q) als Additive in Beschichtungen und Lacken Einsatz finden. Zusätze der Copolymere(Q) zu z. B. strahlungs- oder additionshärtenden Lacken führen zur Verringerung der Oberflächenrauhigkeit und somit zu einer Verringerung des Gleitwiderstands des Lacks. Weiterhin können die Copolymeren (Q) als Zusätze in kosmetischen Formulierungen, beispielsweise als Konditionierer in Haarwaschmitteln, und als Bautenschutzmittel dienen.

Zusätzlich stellen die Copolymere (Q) oberflächenaktive Mittel dar und können als Detergentien, Tenside, Emulgatoren, Entschäumer und Schaumstabilisatoren eingesetzt werden.

Alle vorstehenden Symbole der vorstehenden Formeln weisen ihre Bedeutungen jeweils unabhängig voneinander auf. In allen Formeln ist das Siliciumatom vierwertig.

**Beispiele:**

In den nachfolgenden Beispielen beziehen sich alle Angaben von Teilen und Prozentsätzen, soweit nicht anders angegeben ist, auf das Gewicht. Sofern nicht anders angegeben, werden die nachfolgenden Beispiele bei einem Druck der umgebenden Atmosphäre, also bei etwa 1000 hPa, und bei Raumtemperatur, also bei etwa 20 °C bzw. bei einer Temperatur, die sich beim Zusammengeben der Reaktanden bei Raumtemperatur ohne zusätzliche Heizung und Kühlung einstellt, durchgeführt. Alle in den Beispielen angeführten Viskositätsangaben beziehen sich auf eine Temperatur von 25 °C.

AZ bedeutet Aminzahl, wobei unter Aminzahl die Zahl verstanden werden soll, die der Anzahl von ml 1N HCl entspricht, die zur Neutralisation von 1 g Substanz erforderlich ist.

**Beispiel 1:**

250 g eines endständig aminopropyl-funktionellen Siliconöls (AZ = 0,18) werden mit 4,85 g einer 70%igen wässrigen Lösung von 2,3-Epoxypropyltrimethylammoniumchlorid (QUAB®151, Degussa AG) in 4 g i-Propanol für 12 h bei 50°C gerührt. Anschließend gibt man 25,69 g eines $\alpha$-Acrylat- und $\omega$-Methylether-funktionalisierten Oligoethylenglykols (Mn = ca. 1142) in 250 g i-Propanol zu und rührt für 2 h unter Rückflußbedingungen. Nach Entfernen des Lösungsmittels im Vakuum erhält man 279 g eines farblosen talgartigen Feststoffes (AZ = 0,16).

**Beispiel 2:**

250 g eines endständig aminopropyl-funktionellen Siliconöls (AZ = 0,26) werden mit 14,02 einer 70%igen wässrigen Lösung von 2,3-Epoxypropyltrimethylammoniumchlorid (QUAB®151, Degussa AG) in 10 g i-Propanol für 12 h bei 50°C gerührt. Anschließend gibt man 143 g eines α-Glycidoxy- und ω-Butylether-funktionalisierten Polyethylenglykol-polypropylenglykol-Copolymers (Mn = ca. 2200; ca. 22 EO-Einheiten und 19 PO-Einheiten) in 250 g i-Propanol zu und rührt für weitere 6 h unter Rückflußbedingungen. Nach Entfernen des Lösungsmittels im Vakuum erhält man 302,5 g eines farblosen Feststoffes (AZ = 0,21).

**Beispiel 3:**

250 g eines endständig aminopropyl-funktionellen Siliconöls (AZ = 0,56) werden mit 15,1 g einer 70%igen wässrigen Lösung von 2,3-Epoxypropyltrimethylammoniumchlorid (QUAB®151, Degussa AG) in 10,6 g i-Propanol für 12 h bei 50°C gerührt. Anschließend gibt man 67 g eines α-Glycidoxy- und ω-Butylether-funktionalisierten Polyethylenglykols (Mn = ca. 319) in 250 g 1-Propanol zu und rührt für weitere 6 h unter Rückflußbedingungen. Nach Entfernen des Lösungsmittels im Vakuum erhält man 327,5 g eines weißen wachsartigen Feststoffes (AZ = 0,42).

**Beispiel 4:**

250 g eines α-aminopropyl- und ω-i-butoxyfunktionellen Siliconöls (AZ = 0,5) werden mit 27 g einer 70%igen wässrigen Lösung von 2,3-Epoxypropyltrimethylammoniumchlorid (QUAB°151, Degussa AG) in 19 g i-Propanol für 12 h bei 50°C gerührt.

Anschließend gibt man 142,75 g eines α-Acrylat- und ω-Methylether-funktionalisierten Oligoethylenglykols (Mn = ca. 1142) in 250 g i-Propanol zu und rührt für 4 h unter Rückflußbedingungen. Nach Entfernen des Lösungsmittels im Vakuum erhält man 412 g eines leicht gelbstichigen Feststoffes (AZ = 0,30).

**Beispiel 5:**

250 g eines aminoethylaminopropylfunktionalisierten Siliconöls mit Trimethylsilylendgruppen (AZ = 0,25) werden mit 13,48 g einer 70%igen wässrigen Lösung von 2,3-Epoxypropyltrimethylammoniumchlorid (QUAB°151, Degussa AG) in 9,5 g i-Propanol für 12 h bei 50°C gerührt. Anschließend gibt man 25,2 g eines α-Acrylat- und ω-Methylether-funktionalisierten Oligoethylenglykols (Mn = ca. 807) in 250 g i-Propanol zu und rührt für 3 h unter Rückflußbedingungen. Nach Entfernen des Lösungsmittels im Vakuum erhält man 284,5 g eines leicht gelbstichigen Feststoffes (AZ = 0,22).

**Beispiel 6:**

250 g eines aminoethylaminopropylfunktionalisierten Siliconöls mit Trimethylsilylendgruppen (AZ = 0,60) werden mit 64,13 g einer wässrigen Lösung von 2,3-Epoxypropyldimethyldodecylammoniumchlorid (QUAB°342, Degussa AG) und 4,35 g KOH in 100 g Ethanol für 12 h bei 70°C gerührt. Anschließend gibt man 75,8 g eines α-Glycidoxy- und ω-Butylether-funktionalisierten Polyethylenglykols (Mn = ca. 1010) und 150 g i-Propanol zu und rührt für weitere 8 h unter Rückflußbedingungen. Nach Entfernen des Lösungsmittels im Vakuum erhält man 351 g eines weißen wachsartigen Feststoffes (AZ = 0,43).

**Beispiel 7:**

250 g eines endständig aminopropyl-funktionellen Siliconöls (AZ = 0,18) werden mit 9,7 g einer 70%igen wässrigen Lösung von 2,3-Epoxypropyltrimethylammoniumchlorid (QUAB° 151, Degussa AG) in 7 g i-Propanol für 12 h bei 50°C gerührt. Anschließend gibt man 12,45 g eines α,ω-Diacrylat-funktionalisierten Oligoethylenglykols (Mn = ca. 830) und 34,2 g eines α-Acrylatund ω-Methylether-funktionalisierten Oligoethylenglykols (MW = ca. 1142) in 250 g i-Propanol zu und rührt für 4 h unter Rückflußbedingungen. Nach Entfernen des Lösungsmittels im Vakuum erhält man 303 g eines schwach gelbstichigen, talgartigen Feststoffes (AZ = 0,15).

**Beispiel 8:**

250 g eines endständig aminopropyl-funktionellen Siliconöls (AZ = 0,18) werden mit 9,7 g einer 70%igen wässrigen Lösung von 2,3-Epoxypropyltrimethylammoniumchlorid (QUAB° 151, Fa. degussa) in 7 g i-Propanol für 12 h bei 50°C gerührt. Anschließend gibt man 38 g eines α,ω-Diacrylat-funktionalisierten Oligoethylenglykols (Mn = ca.

1126) in 250 g i-Propanol zu und rührt für 2 h unter Rückflußbedingungen. Nach Entfernen des Lösungsmittels im Vakuum erhält man 295 g eines schwach gelbstichigen, talgartigen Feststoffes (AZ = 0,15).

**Anwendungsbeispiele:**

Foulardverfahren:

Verwendet wurde eine gebleichte, unausgerüstete Webware PES/CO 65/35 Twill mit einem Flächengewicht von 200 $g/m^2$. Als Referenz diente eine Ausrüstung mit einer Standard-Silicon-Weichmacheremulsion (Micro-Emulsion eines aminofunktionellen Polydimethylsiloxans) sowie mit Wasser geklotzte und getrocknete Ware.
Der Stoff wurde mit der jeweiligen Flotte getränkt, mit einem Zweiwalzenfoulard auf 70 % Flottenaufnahme abgequetscht, aufgespannt und in einem Mathis-Laborspannrahmen bei 150 °C 2 min getrocknet. Anschließend wurde die Ware mindestens 12 Stunden im Klimaraum bei 23°C und 50 % Luftfeuchtigkeit klimatisiert.

Bestimmungsmethoden für die Ergebnisse der Anwendungsbeispiele:

A) Bestimmung des Weichgriffs (Griffbewertung)

Da der Weichgriff von Textilien stark dem subjektiven Empfinden der Testpersonen unterliegt, kann nur eine Standardisierung der Randbedingungen, nicht aber der Bewertung erreicht werden. Um trotzdem eine Reproduzierbarkeit zu gewährleisten, wurden die ausgerüsteten Muster hinsichtlich ihres Weichgriffs beurteilt und in eine Rangfolge gebracht. Dazu wurden von 10 Personen in Abhängigkeit der Anzahl n der getesteten Muster 1 bis n Punkte vergeben, wobei n Punkte für das weicheste Muster und 1 Punkt für das am wenigsten weiche Muster vergeben wurden. Die Griffbewertung eines Musters errechnet sich somit als Mittelwert der jeweils auf dieses Muster entfallenen Punkte.

B) Bestimmung der Tropfeneinsinkzeit

Das ausgerüstete Muster wurde nach der Ausrüstung acht Stunden zur Akklimatisation in einem Klimaraum bei einer Temperatur von 23°C und einer Luftfeuchtigkeit von 50 % gelagert, dann wurde ein Tropfen entionisiertes Wasser aus einer Höhe von 6 cm auf die gespannte Stoffoberfläche gegeben und die Zeit bestimmt, nach der der Wassertropfen vom Stoff aufgesaugt war, längstens jedoch drei Minuten. Es wurden fünf Bestimmungen durchgeführt und der Mittelwert gebildet.
In Tabelle 1 sind für einige Anwendungsbeispiele die Ergebnisse des mittels Foulardverfahren ausgerüsteten Stoffs zusammengestellt.

Tabelle 1:

| Beispiele | 1 | 2 | 3 | 4 | 5 | 6 | 7 | 8 | 9 | 10 |
|---|---|---|---|---|---|---|---|---|---|---|
| Emulsion aus Beispiel 1 | 20 g/l | | | | | | | | | |
| Emulsion aus Beispiel 2 | | 20 g/l | | | | | | | | |
| Emulsion aus Beispiel 3 | | | 20 g/l | | | | | | | |
| Emulsion aus Beispiel 4 | | | | 20 g/l | | | | | | |
| Emulsion aus Beispiel 5 | | | | | 20 g/l | | | | | |
| Emulsion aus Beispiel 6 | | | | | | 20 g/l | | | | |
| Emulsion aus Beispiel 7 | | | | | | | 20 g/l | | | |
| Emulsion aus Beispiel 8 | | | | | | | | 20 g/l | | |

Tabelle 1: (fortgesetzt)

| Beispiele | 1 | 2 | 3 | 4 | 5 | 6 | 7 | 8 | 9 | 10 |
|---|---|---|---|---|---|---|---|---|---|---|
| Standard Si-Micro Emulsion | | | | | | | | | 20 g/l | |
| Eisessig | 0,5 g/l | 0,5 g/l | 0,5 g/l | 0,5 g/l | 0,5 g/l | 0,5 g/l | 0,5 g/l | 0,5 g/l | 0,5 g/l | 0,5 g/l |
| Tropfeneinsinkzeit (in s) | 16 | <1 | <1 | <1 | 130 | 54 | 8 | 13 | >180 | 108 |
| Griff | 7,1 | 2,1 | 3,6 | 1,9 | 6,3 | 3,8 | 7,9 | 8,7 | 3,6 | 0 |

**Patentansprüche**

**1.** Copolymere (Q) der allgemeinen Formel (I)

$$A\text{-}B\text{-}(D\text{-}B)_o\text{-}A \quad (I),$$

worin,

**A** einen Rest $-R^1$ oder $-OR^1$ oder einen einwertigen Rest der allgemeinen Formel (II),

**A** einen Rest $-R^1$ oder $-OR^1$ oder einen einwertigen Rest der allgemeinen Formel (II),

$$-R\text{-}\left[(NH_aE_bF_c)^{(a+2b+c-1)+}\text{-}R\right]_g\text{-}(NH_dE_eF_f)^{(d+2e+f-2)+}\ [g(a+2b+c-1)+d+2e+f-2]\ X^- \quad (II),$$

**$R^1$** ein Wasserstoffatom oder einen einwertigen Kohlenwasserstoffrest mit 1 bis 100 Kohlenstoffatomen, der gegebenenfalls durch die Atome N, O, P, B, Si, S unterbrochen oder substituiert sein kann, oder Einheiten -C(O)-, -C(O)O-, - C(O)$NR^9$-, -$NR^9$-, -O-, -S-, =N-, =N enthalten kann, oder durch Gruppen -$NR^9$-, -OH, -SH substituiert sein kann,

**R** einen zweiwertigen Kohlenwasserstoffrest mit 1 bis 50 Kohlenstoffatomen, der gegebenenfalls durch die Atome N, O, P, B, Si, S unterbrochen oder substituiert sein kann, oder Einheiten -C(O)-, -C(O) O-, -C(O)$NR^9$-, -$NR^9$-, -O-, -S-, =N- enthalten kann,

**$R^9$** ein Wasserstoffatom oder einen einwertigen Kohlenwasserstoffrest mit 1 bis 18 Kohlenstoffatomen, der gegebenenfalls durch die Atome N, O, P, B, Si, S unterbrochen oder oder durch Gruppen -OH, -SH substituiert sein kann,

**a, b** und **c** ganze Zahlen von 0 bis 2, wobei die Summe aus **a+b+c** gleich 1 oder 2 ist,

**d, e** und **f** ganze Zahlen von 0 bis 3, wobei die Summe aus **d+e+f** gleich 2 oder 3 ist,

**g** eine ganze Zahl von 0 bis 10,

**E** einen Rest der allgemeinen Formel (III)

$$-R^2\text{-}NR^3{}_3{}^+ \quad (III),$$

**$R^2$** einen zweiwertigen Kohlenwasserstoffrest mit 1 bis 10 Kohlenstoffatomen, der gegebenenfalls durch die Atome N, O, P, B, Si, S unterbrochen oder substituiert sein kann, oder Einheiten -C(O)-, -C(O) O-, -C (O) $NR^9$-, -$NR^9$-, -O-, -S-, =N-, ≡N enthalten kann, oder durch Gruppen -$NR^9$-, -OH, -SH substituiert sein kann,

**$R^3$** einen einwertigen Kohlenwasserstoffrest mit 1 bis 20 Kohlenstoffatomen, der gegebenenfalls durch die Atome N, O, P, B, Si, S unterbrochen oder substituiert sein kann, oder Einheiten -C(O)-, -C(O)

O-, - -C (O) $NR^9$-, -$NR^9$-, -O-, -S-, =N-, ≡N enthalten kann, oder durch Gruppen -$NR^9$-, -OH, -SH substituiert sein kann,

**$X^-$** ein anorganisches oder organisches Anion,

**F** einen Rest der allgemeinen Formeln (IV) oder (V)

$$-CH_2-CHR^4-C(O)-G-(R^2-G)_h-[CH_2CH_2O]_i-[C_3H_6O]_j-[(CH_2)_4O]_k-(R^2-G)_h-R^5 \quad (IV),$$

$$-CH_2-CH(OH)-CH_2-G-(R^2-G)_h-[CH_2CH_2O]_i-[C_3H_6O]_j-[(CH_2)_4O]_k-(R^2-G)_h-R^5 \quad (V),$$

**$R^4$** ein Wasserstoffatom oder Methylrest,

**$R^5$** ein Wasserstoffatom, ein einwertiger, verzweigter oder unverzweigter Kohlenwasserstoffrest mit 1 bis 10 Kohlenstoffatomen oder eine Einheit ausgewählt aus der Gruppe $CH_2=CR^4-C(O)-$, $(R^1)_2N-CH_2-CHR^4-C(O)-$, $R^4-CH_2-C(O)-$, $HO-CH_2-CH(OH)-CH_2-$ oder

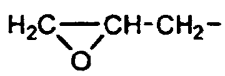

,

**G** eine zweiwertige Gruppe -O- oder -$NR^9$-,

**h** den Wert 0 oder 1,

**i, j** und **k** jeweils eine ganze Zahl von 0 bis 200,

**B** einen zweiwertigen Rest, aufgebaut aus Einheiten, die ausgewählt werden aus den allgemeinen Formeln (VIa), (VIb), (VIc), (VId) und A

$$(SiR^6{}_2O_{2/2})_1 \quad (VIa),$$

$$(SiR^6R^7O_{2/2})_m \quad (VIb),$$

$$(-SiR^6{}_2-)_{p+1} \quad (VIc),$$

$$(SiO_{3/2})_p \quad (VId),$$

**$R^6$** ein Wasserstoffatom, -$OR^1$, oder einen einwertigen Alkylrest mit 1 bis 200 Kohlenstoffatomen, der substituiert sein kann durch Halogenatome, Carboxy-, Epoxy-, Hydroxy- oder Polyethergruppen und gegebenenfalls durch die Einheiten -C(O)-, -C(O)O-, -C(O)$NR^9$-, -$NR^9$-, -O-, -S-, unterbrochen sein kann, bedeutet,

**$R^7$** ein einwertiger Rest der allgemeinen Formel (II),

**l** und **m** eine ganze Zahl von 0 bis 5000,

**p** eine ganze Zahl von 0 bis 500,

**D** einen zweiwertigen Rest der allgemeinen Formel (VIII),

$$-\{[R-(NH_aE_bF_c)]_n-J-[(NH_aE_bF_c)-R]_n\}-^{[2n(a+2b+c)-4]+} \quad [2n(a+2b+c)-4]\ X^- \quad (VIII),$$

**n** eine ganze Zahl von 1 bis 10,

**J** einen zweiwertigen Rest der allgemeinen Formeln (IX) oder (X),

$$-CH_2-CHR^4-C(O)-G-(R^2-G)_h-[CH_2CH_2O]_i-[C_3H_6O]_j-[(CH_2)_4O]_k-(R^2-G)_h-$$

$$C(O)\text{-}CHR^4\text{-}CH_2\text{-} \qquad (IX),$$

$$\text{-}CH_2\text{-}CH(OH)\text{-}CH_2\text{-}G\text{-}(R^2\text{-}G)_h\text{-}[CH_2CH_2O]_i\text{-}[C_3H_6O]_j\text{-}[(CH_2)_4O]_k\text{-}(R^2\text{-}G)_h\text{-}CH_2\text{-}CH(OH)\text{-}CH_2\text{-} \qquad (X),$$

**o** eine ganze Zahl ≥ 0 bedeuten,

mit der Maßgabe, daß die Copolymere der allgemeinen Formel (I) mindestens je einen Rest **E** und **F** enthalten.

**2.** Copolymere (Q) nach Anspruch 1, welche ein durchschnittliches Molekulargewicht Mn von 500 g/mol bis 1 000 000 g/mol besitzen.

**3.** Copolymere (Q) nach Anspruch 1 oder 2, bei denen **R** einen Rest-$(CH_2)$-, -$(CH_2)_2$- oder -$(CH_2)_3$- bedeutet.

**4.** Copolymere (Q) nach Anspruch 1 bis 3, bei denen $\mathbf{R^1}$ Wasserstoffatom, Methyl-, Ethyl-, Propyl-, Cyclohexyl- oder der Acetylrest bedeutet.

**5.** Copolymere (Q) nach Anspruch 1 bis 4, bei denen $\mathbf{R^2}$ einen linearen oder verzweigten Alkylenrest bedeutet.

**6.** Verfahren zur Herstellung von Copolymeren (Q) der allgemeinen Formel (I) gemäss Anspruch 1, bei dem Organosiliciumverbindungen oder Mischungen davon, die ausgewählt werden aus Verbindungen der allgemeinen Formeln (XI), (XII), (XIII), (XIV) und (XV)

$$H\text{-}NR^1\text{-}(R\text{-}NHR^1)_g\text{-}R\text{-}(SiR^6{}_2O)_1\text{-}SiR^6{}_2\text{-}R(\text{-}NHR^1\text{-}R)_g\text{-}NR^1\text{-}H \qquad (XI),$$

$$H\text{-}NR^1\text{-}(R\text{-}NHR^1)_g\text{-}R\text{-}(SiR^6{}_2O)_1\text{-}\{SiR^6[\text{-}R(\text{-}NHR^1\text{-}R)_g\text{-}NHR^1]\text{-}O\}_m\text{-}R(\text{-}NHR^1\text{-}R)_g\text{-}NR^1\text{-}H \qquad (XII),$$

$$R^6{}_3SiO\text{-}(SiR^6{}_2O)_1\text{-}\{SiR^6[\text{-}R(\text{-}NHR^1\text{-}R)_g\text{-}NHR^1]\text{-}O\}_m\text{-}SiR^6{}_3 \qquad (XIII),$$

$$MeO\text{-}SiR^6{}_2O\text{-}(SiR^6{}_2O)_1\text{-}\{SiR^6[\text{-}R(\text{-}NHR^1\text{-}R)_g\text{-}NHR^1]\text{-}O\}_m\text{-}SiR^6{}_2\text{-}OMe \qquad (XIV),$$

$$H\text{-}NR^1\text{-}(R\text{-}NHR^1)_g\text{-}R\text{-}(SiR^6{}_2O)_1\text{-}SiR^6{}_2\text{-}R^8 \qquad (XV),$$

mit Polyethern, die ausgewählt werden aus Verbindungen der allgemeinen Formeln (XVI) und (XVII)

$$CH_2=CH\text{-}C(O)\text{-}G\text{-}(R^2\text{-}G)_h\text{-}[CH_2CH_2O]_i\text{-}[C_3H_6O]_j\text{-}[(CH_2)_4O]_k\text{-}(R^2\text{-}G)_h\text{-}R^5 \qquad (XVI),$$

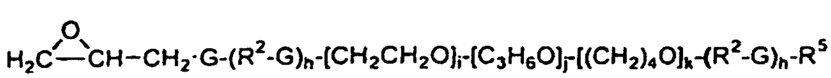

(XVII)

und organischen Verbindungen, die ausgewählt werden aus Verbindungen der allgemeinen Formeln (XVIII) und (XIX)

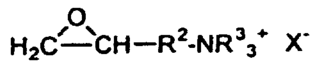

(XVIII)

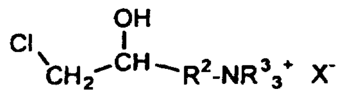

(XIX)

umgesetzt werden, wobei **Me** Methylrest bedeutet und **$R^1$, $R^2$, $R^3$, $R^5$, $R^6$, G, X, g, h, i, j, k, l,** sowie m die in Anspruch 1 dafür angegebenen Bedeutungen aufweisen.

## Claims

**1.** Copolymers (Q) of the general formula (I)

$$A\text{-}B\text{-}(D\text{-}B)_o\text{-}A \qquad (I),$$

where

**A** is an $-R^1$ or $-OR^1$ radical or a monovalent radical of the general formula (II),

$$-R\text{-}[(NH_aE_bF_c)^{(a+2b+c-1)+}\text{-}R]_g\text{-}(NH_dE_eF_f)^{(d+2e+f-2)+}\ [g(a+2b+c-1)+d+2e+f-2]\ X^- \qquad (II),$$

**$R^1$** is a hydrogen atom or a monovalent hydrocarbyl radical having 1 to 100 carbon atoms that may optionally be interrupted or substituted by the atoms N, 0, P, B, Si, S or contain -C(O)-, -C(O)O-, -C (O)$NR^9$-, -$NR^9$-, -O-, -S-, =N-, ≡N units or be substituted by -$NR^9$-, -OH, -SH groups,

**R** is a divalent hydrocarbyl radical having 1 to 50 carbon atoms that may optionally be interrupted or substituted by the atoms N, 0, P, B, Si, S or contain -C(O)-, -C(O)O-, -C(O)$NR^9$-, -$NR^9$-, -O-, -S-, =N- units,

**$R^9$** is a hydrogen atom or a monovalent hydrocarbyl radical having 1 to 18 carbon atoms that may optionally be interrupted by the atoms N, 0, P, B, Si, S or substituted by -OH, -SH groups,

**a, b** and **c** are integers from 0 to 2 subject to the condition that the sum total of **a+b+c** is 1 or 2,

**d, e** and **f** are integers from 0 to 3 subject to the condition that the sum total of **d+e+f** is 2 or 3,

**g** is an integer from 0 to 10,

**E** is a radical of the general formula (III)

$$-R^2\text{-}NR^3{}_3{}^+ \qquad (III),$$

**$R^2$** is a divalent hydrocarbyl radical having 1 to 10 carbon atoms that may optionally be interrupted or substituted by the atoms N, O, P, B, Si, S or contain -C(O)-, -C(O)O-, -C(O)$NR^9$-, -$NR^9$-, -O-, -S-, =N-, ≡N units or be substituted by -$NR^9$-, -OH, -SH groups,

**$R^3$** is a monovalent hydrocarbyl radical having 1 to 20 carbon atoms that may optionally be interrupted or substituted by the atoms N, 0, P, B, Si, S or contain -C(O)-, -C(O)O-, - -C(O)$NR^9$-, -$NR^9$-, -O-, -S-, =N-, ≡N units or be substituted by -$NR^9$-, -OH, -SH groups,

**$X^-$** is an organic or inorganic anion,

**F** is a radical of the general formulae (IV) or (V)

$$-CH_2\text{-}CHR^4\text{-}C(O)\text{-}G\text{-}(R^2\text{-}G)_h\text{-}[CH_2CH_2O]_i\text{-}[C_3H_6O]_j\text{-}[(CH_2)_4O]_k\text{-}(R^2\text{-}G)_h\text{-}R^5 \qquad (IV),$$

$$-CH_2-CH(OH)-CH_2-G-(R^2-G)_h-[CH_2CH_2O]_i-[C_3H_6O]_j-[(CH_2)_4O]_k-(R^2-G)_h-R^5 \qquad (V),$$

**$R^4$** is a hydrogen atom or methyl radical,

**$R^5$** is a hydrogen atom, a monovalent branched or unbranched hydrocarbyl radical having 1 to 10 carbon atoms or a unit selected from the group consisting of $CH_2=CR^4-C(O)-$, $(R^1)_2N-CH_2-CHR^4-C(O)-$, $R^4-CH_2-C(O)-$, $HO-CH_2-CH(OH)-CH_2-$ and

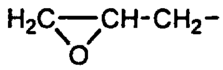
,

**G** is a divalent group -O- or $-NR^9-$,

**h** is 0 or 1,

**i, j** and **k** are each an integer from 0 to 200,

**B** is a divalent radical constructed from units which are selected from the general formulae (VIa), (VIb), (VIc), (VId) and **A**

$$(SiR^6{}_2O_{2/2})_1 \qquad (VIa),$$

$$(SiR^6R^7O_{2/2})_m \qquad (VIb),$$

$$(-SiR^6{}_2-)_{p+1} \qquad (VIc),$$

$$(SiO_{3/2})_p \qquad (VId),$$

**$R^6$** is a hydrogen atom, $-OR^1$, or a monovalent alkyl radical having 1 to 200 carbon atoms which may be substituted by halogen atoms, carboxyl groups, epoxy groups, hydroxyl groups or polyether groups and may optionally be interrupted by the units -C(O)-, -C(O)O-, $-C(O)NR^9-$, $-NR^9-$, -O-, -S-,

**$R^7$** is a monovalent radical of the general formula (II),

**1** and **m** are an integer from 0 to 5000,

p is an integer from 0 to 500,

**D** is a divalent radical of the general formula (VIII),

$$-\{[R-(NH_aE_bF_c)]_n-J-[(NH_aE_bF_c)-R]_n\}^{-[2n(a+2b+c)-4]-}\ [2n(a+2b+c)-4]\ X^- \qquad (VIII),$$

**n** is an integer from 1 to 10,

**J** is a divalent radical of the general formulae (IX) or (X),

$$-CH_2-CHR^4-C(O)-G-(R^2-G)_h-[CH_2CH_2O]_i-[C_3H_6O]_j-[(CH_2)_4O]_k-(R^2-G)_h-C(O)-CHR^4-CH_2- \qquad (IX),$$

$$-CH_2-CH(OH)-CH_2-G-(R^2-G)_h-[CH_2CH_2O]_i-[C_3H_6O]_j-[(CH_2)_4O]_k-(R^2-G)_h-CH_2-CH(OH)-CH_2- \qquad (X),$$

**o** is an integer ≥ 0,

with the proviso that the copolymers of the general formula (I) contain at least one **E** radical and one **F** radical.

**2.** Copolymers (Q) according to Claim 1 having an average molecular weight Mn from 500 g/mol to 1 000 000 g/mol.

**3.** Copolymers (Q) according to Claim 1 or 2 wherein **R** is a $-(CH_2)-$, $-(CH_2)_2-$ or $-(CH_2)_3-$ radical.

**4.** Copolymers (Q) according to Claim 1 to 3 wherein $\mathbf{R^1}$ is hydrogen, methyl, ethyl, propyl, cyclohexyl or acetyl.

**5.** Copolymers (Q) according to Claim 1 to 4 wherein $\mathbf{R^2}$ is a linear or branched alkylene radical.

**6.** Process for preparing copolymers (Q) of the general formula (I) as per Claim 1, which comprises reacting organosilicon compounds or mixtures thereof that are selected from compounds of the general formulae (XI), (XII), (XIII), (XIV) and (XV)

$$H\text{-}NR^1\text{-}(R\text{-}NHR^1)_g\text{-}R\text{-}(SiR^6{}_2O)_1\text{-}SiR^6{}_2\text{-}R(\text{-}NHR^1\text{-}R)_g\text{-}NR^1\text{-}H \qquad (XI),$$

$$H\text{-}NR^1\text{-}(R\text{-}NHR^1)_g\text{-}R\text{-}(SiR^6{}_2O)_1\text{-}\{SiR^6[\text{-}R(\text{-}NHR^1\text{-}R)_g\text{-}NHR^1]\text{-}O\}_m\text{-}R(\text{-}NHR^1\text{-}R)_g\text{-}NR^1\text{-}H \qquad (XII),$$

$$R^6{}_3SiO\text{-}(SiR^6{}_2O)_1\text{-}\{SiR^6[\text{-}R(\text{-}NHR^1\text{-}R)_g\text{-}NHR^1]\text{-}O\}_m\text{-}SiR^6{}_3 \qquad (XIII),$$

$$MeO\text{-}SiR^6{}_2O\text{-}(SiR^6{}_2O)_1\text{-}\{SiR^6[\text{-}R(\text{-}NHR^1\text{-}R)_g\text{-}NHR^1]\text{-}O\}_m\text{-}SiR^6{}_2\text{-}OMe \qquad (XIV),$$

$$H\text{-}NR^1\text{-}(R\text{-}NHR^1)_g\text{-}R\text{-}(SiR^6{}_2O)_1\text{-}SiR^6{}_2\text{-}R^8 \qquad (XV),$$

with polyethers which are selected from compounds of the general formulae (XVI) and (XVII)

$$CH_2{=}CH\text{-}C(O)\text{-}G\text{-}(R^2\text{-}G)_h\text{-}[CH_2CH_2O]_i\text{-}[C_3H_6O]_j\text{-}[(CH_2)_4O]_k\text{-}(R^2\text{-}G)_h\text{-}R^5 \qquad (XVI),$$

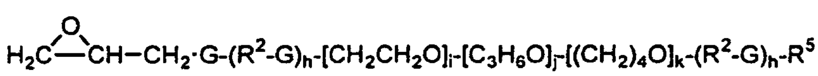
(XVII)

and organic compounds which are selected from compounds of the general formulae (XVIII) and (XIX)

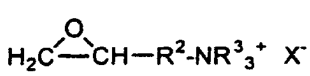
(XVIII)

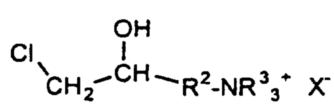
(XIX)

where **Me** is methyl and $\mathbf{R^1}$, $\mathbf{R^2}$, $\mathbf{R^3}$, $\mathbf{R^5}$, $\mathbf{R^6}$, **G, X, g, h, i, j, k, 1** and **m** are each as defined in Claim 1.

## Revendications

**1.** Copolymères (Q) de formule générale (I)

$$A\text{-}B\text{-}(D\text{-}B)_o\text{-}A \qquad (I)$$

dans laquelle

**A** représente un radical $-R^1$ ou $-OR^1$ ou un radical monovalent de formule générale (II)

$$-R\text{-}[\,(NH_aE_bF_c)^{(a+2b+c-1)+}\text{-}R]_g\text{-}(NH_dE_eF_f)^{(d+2e+f-2)+}$$

$$[g(a+2b+c-1)+d+2e+f-2]\ X^- \qquad (II)$$

**$R^1$** représente un atome d'hydrogène ou un radical hydrocarboné monovalent ayant de 1 à 100 atomes de carbone, qui éventuellement peut être interrompu ou substitué par les atomes N, O, P, B, Si, S, ou peut contenir des motifs -C(O)-, -C(O)O-, $-C(O)NR^9-$, $-NR^9-$, -O-, -S-, =N-, ≡N, ou peut être substitué par des groupes $-NR^9-$, -OH, -SH,

**R** représente un radical hydrocarboné divalent ayant de 1 à 50 atomes de carbone, qui éventuellement peut être interrompu ou substitué par les atomes N, O, P, B, Si, S, ou peut contenir des motifs -C(O)-, -C(O)O-, $-C(O)NR^9-$, $-NR^9-$, -O-, -S-, =N-,

**$R^9$** représente un atome d'hydrogène ou un radical hydrocarboné monovalent ayant de 1 à 18 atomes de carbone, qui peut éventuellement être interrompu par les atomes N, 0, P, B, Si, S, ou substitué par des groupes -OH, -SH,

a, b et c représentent des nombres entiers allant de 0 à 2, la somme de a + b + c étant égale à 1 ou 2,

d, e et f représentent des nombres entiers allant de 0 à 3, la somme de d + e + f étant égale à 2 ou 3,

**g** représente un nombre entier allant de 0 à 10,

**E** représente un radical de formule générale (III)

$$-R^2\text{-}NR^3{}_3{}^+ \qquad (III)$$

**$R^2$** représente un radical hydrocarboné divalent ayant de 1 à 10 atomes de carbone, qui éventuellement peut être interrompu ou substitué par les atomes N, 0, P, B, Si, S, ou peut contenir des motifs -C(O)-, -C(O)O-, $-C(O)NR^9-$, $-NR^9-$, -O-, -S-, =N-, ≡N, ou peut être substitué par des groupes $-NR^9-$, -OH, -SH,

**$R^3$** représente un radical hydrocarboné monovalent ayant de 1 à 20 atomes de carbone, qui éventuellement peut être interrompu ou substitué par les atomes N, O, P, B, Si, S, ou peut contenir des motifs -C(O)-, -C(O)O-, $-C(O)NR^9-$, $-NR^9-$, -O-, -S-, =N-, ≡N, ou peut être substitué par des groupes $-NR^9-$, -OH, -SH,

**$X^-$** représente un anion organique ou minéral,

**F** représente un radical de formule générale (IV) ou (V)

$$-CH_2\text{-}CHR^4\text{-}C(O)\text{-}G\text{-}\,(R^2\text{-}G)_h\text{-}[CH_2CH_2O]_i\text{-}[C_3H_6O]_j\text{-}$$

$$[(CH_2)_4O]_k\text{-}(R^2\text{-}G)_h\text{-}R^5 \qquad (IV),$$

$$-CH_2\text{-}CH(OH)\text{-}CH_2\text{-}G\text{-}(R^2\text{-}G)_h\text{-}[CH_2CH_2O]_i\text{-}[C_3H_6O]_j\text{-}$$

$$[(CH_2)_4O]_k\text{-}(R^2\text{-}G)_h\text{-}R^5 \qquad (V),$$

**$R^4$** représente un atome d'hydrogène ou le radical méthyle,

**$R^5$** représente un atome d'hydrogène, un radical hydrocarboné monovalent, ramifié ou non ramifié, ayant de 1 à 10 atomes de carbone, ou un motif choisi dans l'ensemble constitué par $CH_2=CR^4-C(O)-$, $(R^1)_2N-CH_2-CHR^4-C(O)-$, $R^4-CH_2-C(O)-$, $HO-CH_2-$, $CH(OH)-CH_2-$ ou

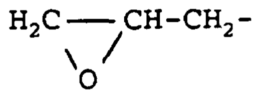

**G** représente un groupe divalent -O- ou $-NR^9-$,
**h** a la valeur 0 ou 1,
**i, j** et **k** représentent chacun un nombre entier allant de 0 à 200,
**B** représente un radical divalent, constitué de motifs qui sont choisis parmi les formules générales (VIa), (VIb), (VIc), (VId) et **A**

$$(SiR^6{}_2O_{2/2})_1 \quad \text{(VIa)},$$

$$(SiR^6R^7O_{2/2})_m \quad \text{(VIb)},$$

$$(-SiR^6{}_2-)_{p+1} \quad \text{(VIc)},$$

$$(SiO_{3/2})_p \quad \text{(VId)},$$

**$R^6$** représente un atome d'hydrogène, $-OR^1$, ou un radical alkyle monovalent ayant de 1 à 200 atomes de carbone, qui peut être substitué par des atomes d'halogène, des groupes carboxy, époxy, hydroxy ou polyéther, et peut éventuellement être interrompu par les motifs -C(O)-, -C(O)O-, $-C(O)NR^9-$, $-NR^9-$, -O-, -S-,
**$R^7$** représente un radical monovalent de formule générale (II),
1 et m représentent un nombre entier allant de 0 à 5000,
p représente un nombre entier allant de 0 à 500,
D représente un radical divalent de formule générale (VIII)

$$-\{[R-(NH_aE_bF_c)]_n-J-[(NH_aE_bF_c)-R]_n\}-^{[2n(a+2b+c)-4]+}$$
$$[2n(a+2b+c)-4]\ X^- \quad \text{(VIII)},$$

**n** représente un nombre entier allant de 1 à 10,
**J** représente un radical divalent de formule générale (IX) ou (X)

$$-CH_2-CHR^4-C(O)-G-(R^2-G)_h-[CH_2CH_2O]_i-[C_3H_6O]_j-$$
$$[(CH_2)_4O]_k-(R^2-G)_h-C(O)-CHR^4-CH_2- \quad \text{(IX)},$$

$$-CH_2-CH(OH)-CH_2-G-(R^2-G)_h-[CH_2CH_2O]_i-[C_3H_6O]_j-$$
$$[(CH_2)_4O]_k-(R^2-G)_h-CH_2-CH(OH)-CH_2- \quad \text{(X)},$$

**o** représente un nombre entier ≥ 0,

étant entendu que les copolymères de formule générale (I) contiennent chacun au moins un radical **E** et un radical **F**.

**2.** Copolymères (Q) selon la revendication 1, qui ont une masse moléculaire moyenne Mn de 500 g/mole à 1 000 000 g/mole.

**3.** Copolymères (Q) selon la revendication 1 ou 2, dans lesquels **R** représente un radical $-(CH_2)-$, $-(CH_2)_2-$ ou $-(CH_2)_3-$.

**4.** Copolymères (Q) selon l'une quelconque des revendications 1 à 3, dans lesquels $\mathbf{R^1}$ représente un atome d'hydrogène, le radical méthyle, éthyle, propyle, cyclohexyle ou acétyle.

**5.** Copolymères (Q) selon l'une quelconque des revendications 1 à 4, dans lesquels $\mathbf{R^2}$ représente un radical alkylène linéaire ou ramifié.

**6.** Procédé pour la préparation de copolymères (Q) de formule générale (I) selon la revendication 1, dans lequel on fait réagir des composés organosiliciés ou des mélanges de ceux-ci, qui sont choisis parmi les composés de formule générale (XI), (XII), (XIII), (XIV) et (XV)

$$H\text{-}NR^1\text{-}(R\text{-}NHR^1)_g\text{-}R\text{-}(SiR^6{}_2O)_1\text{-}Si^6{}_2\text{-}R(\text{-}NHR^1\text{-}R)_g\text{-}NR^1\text{-}H \quad \text{(XI)},$$

$$H\text{-}NR^1\text{-}(R\text{-}NHR^1)_g\text{-}R\text{-}(SiR^6{}_2O)_1\text{-}\{SiR^6[\text{-}R(\text{-}NHR^1\text{-}R)_g\text{-}NHR^1]\text{-}O\}_m\text{-}R(\text{-}NHR^1\text{-}R)_g\text{-}NR^1\text{-}H \quad \text{(XII)},$$

$$R^6{}_3SiO\text{-}(SiR^6{}_2O)_1\text{-}\{SiR^6[\text{-}R(\text{-}NHR^1\text{-}R)_g\text{-}NHR^1]\text{-}O\}_m\text{-}SiR^6{}_3 \quad \text{(XIII)},$$

$$MeO\text{-}SiR^6{}_2O\text{-}(SiR^6{}_2O)_1\text{-}\{SiR^6[\text{-}R(\text{-}NHR^1\text{-}R)_g\text{-}NHR^1]\text{-}O\}_m\text{-}SiR^6{}_2\text{-}OMe \quad \text{(XIV)},$$

$$H\text{-}NR^1\text{-}(R\text{-}NHR^1)_g\text{-}R\text{-}(Sir^6{}_2O)_1\text{-}SiR^6{}_2\text{-}R^8 \quad \text{(XV)},$$

avec des polyéthers qui sont choisis parmi des composés de formule générale (XVI) et (XVII)

$$CH_2{=}CH\text{-}C(O)\text{-}G\text{-}(R^2\text{-}G)_h\text{-}[CH_2CH_2O]_i\text{-}[C_3H_6O]_j\text{-}[(CH_2)_4O]_k\text{-}(R^2\text{-}G)_h\text{-}R^5 \quad \text{(XVI)},$$

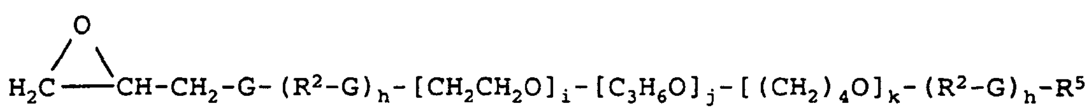

(XVII)

et des composés organiques qui sont choisis parmi des composés de formule générale (XVIII) et (XIX)

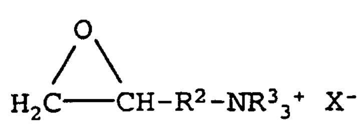

(XVIII)

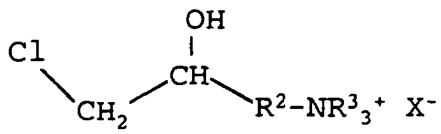

(XIX)

**Me** représentant le radical méthyle et **$R^1$**, **$R^2$**, **$R^3$**, **$R^5$**, **$R^6$**, **G**, **X**, **g**, **h**, **i**, **j**, **k**, **1** ainsi que **m** ayant les significations indiquées pour ceux-ci dans la revendication 1.